# EUROPEAN PATENT APPLICATION

(11) **EP 4 001 258 A1**
(43) Date of publication of application: **25.05.2022**
(21) Application number: 21852024.5
(22) Date of filing: 08.09.2021
(51) Int. Cl.: C07C 69/73, C07C 69/38, C07C 59/90, C07C 59/72, A61K 31/216, A61K 31/225, A61P 29/00

(54) **ARYL PROPIONIC ACID DERIVATIVE, PHARMACEUTICAL COMPOSITION, AND METHOD FOR PREPARATION THEREOF AND APPLICATION THEREOF**

(30) Priority: 09.09.2020 CN 202010943996
(71) Applicant: Nanjing Heron Pharmaceutical Science and Technology Co., Ltd., Nanjing, Jiangsu 211100 (CN)
(72) Inventor: YE, Hai, Nanjing, Jiangsu 211100 (CN); MIN, Tao, Nanjing, Jiangsu 211100 (CN); LV, Tian, Nanjing, Jiangsu 211100 (CN); ZHOU, Wenliang, Nanjing, Jiangsu 211100 (CN); XU, Ying, Nanjing, Jiangsu 211100 (CN); FENG, Yunqing, Nanjing, Jiangsu 211100 (CN)
(74) Representative: HGF
(86) International application number: PCT/CN2021/117108
(87) International publication number: WO 2022/052936

(57) **Abstract**

Provided are an arylpropionic acid derivative represented by Formula (I), a pharmaceutical composition and a preparation method and an application thereof. The arylpropionic acid derivative has a good half-life, pharmacokinetic property and in vitro stability and can enhance efficacy and reduce toxicity after formulated into preparations, which repairs the defects of frequent administration, gastrointestinal side effects and poor patient compliance of traditional nonsteroidal anti-inflammatory drugs.

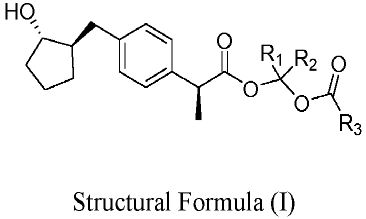

## Description

### CROSS-REFERENCES TO RELATED APPLICATIONS

This application claims priority to Chinese Patent Application No. 202010943996.5 filed with the China National Intellectual Property Administration (CNIPA) on Sep. 9, 2021 and titled *LOXOPROFEN DERIVATIVE, PHARMACEUTICAL COMPOSITION AND PREPARATION METHOD AND APPLICATION THEREOF,* the disclosure of which is incorporated herein by reference in its entirety.

### TECHNICAL FIELD

The present application belongs to the field of medicine and, in particular, relates to an arylpropionic acid derivative, a preparation method and an application thereof, and a pharmaceutical composition containing the arylpropionic acid derivative.

### BACKGROUND

Loxoprofen sodium is a nonsteroidal anti-inflammatory drug based on an arylpropionic acid and developed by Japan Daiichi Sankyo Company. Loxoprofen sodium is non-selective cyclooxygenase inhibitor and achieves anti-inflammatory and analgesic effects by inhibiting arachidonic acid from being catalyzed to prostaglandins and the synthesis of unsaturated fatty acids. At present, there are only oral and topical preparations of loxoprofen sodium in clinics. Oral administration, due to a relatively short half-life, needs to be carried out 3 or 4 times per day and easily causes gastrointestinal injury. Particularly, patients who require long-term administration or patients with gastric ulcers tend to be intolerable to the oral administration.

Loxoprofen sodium is a prodrug. Under the action of carbonyl reductase in a human body, a cyclopentanone group of loxoprofen sodium is stereoselectively reduced to generate eight stereoisomers in theory, an active metabolite of which has the main biological activity, that is, (S)-2-(4-(((1R, 2S)-2-hydroxycyclopentyl)methyl)phenyl)propionic acid (Compound a) with the following structural formula:

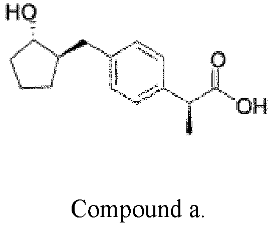

In a medicine development phase, it is found that the active metabolite of loxoprofen is a white solid or an off-white solid in appearance, has low solubility in pure water, and is soluble in a slightly alkaline aqueous solution. The active metabolite has poor physicochemical stability and tends to become viscous and yellow under the conditions of high temperature, high humidity, a cold white fluorescent lamp and an ultraviolet lamp, and solids are easy to absorb moisture and cohere to each other, which makes it difficult to prepare solid preparations. Therefore, it is very necessary to further optimize and modify the structure of the active metabolite of loxoprofen, Compound a.

### SUMMARY

In the present application, a structure of an active metabolite of loxoprofen, Compound a, is further optimized and a carboxyl group in the active metabolite is derivatized so that a series of ester derivatives are designed and prepared, which overcome the defects of poor solubility and stability of Compound a and reduce an administration dosage compared with loxoprofen sodium.

The present application provides an arylpropionic acid derivative, a pharmaceutical composition and a preparation method and an application thereof.

In a first aspect, the present application provides an arylpropionic acid derivative, that is, a compound represented by Formula (I) or a racemate, stereoisomer or pharmaceutically acceptable salt or solvate thereof, 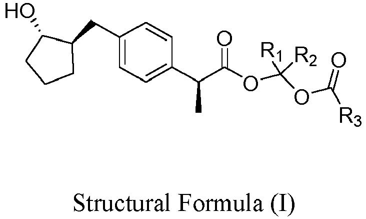 wherein R₁, R₂ and R₃ are the same or different and each independently selected from hydrogen, C₁₋₄₀ alkyl, C₂₋₄₀ alkenyl, C₂₋₄₀ alkynyl, C₁₋₄₀ alkoxy, C₃₋₄₀ cycloalkyl, C₃₋₄₀ cycloalkyloxy, 3- to 20-membered heterocyclic groups, C₆₋₂₀ aryl, 5- to 20-membered heteroaryl or 3- to 20-membered heterocyclic groups substituted with one, two or more Ra; where the one, two or more Ra are the same or different and each independently selected from halogen, C₁₋₄₀ alkyl, C₁₋₄₀ alkoxy or C₆₋₂₀ arylacyl.

According to an embodiment of the present application, R₁, R₂ and R₃ are the same or different and each independently selected from hydrogen, C₁₋₂₀ alkyl, C₂₋₂₀ alkenyl, C₂₋₂₀ alkynyl, C₁₋₂₀ alkoxy, C₃₋₂₀ cycloalkyl, C₃₋₂₀ cycloalkyloxy, 5- to 10-membered heterocyclic groups, C₆₋₁₄ aryl, 5- to 14-membered heteroaryl or 5- to 14-membered heterocyclic groups substituted with one, two or more Ra; where the one, two or more Ra are the same or different and each independently selected from halogen, C₁₋₂₀ alkyl, C₁₋₂₀ alkoxy or C₆₋₂₀ arylacyl.

According to an embodiment of the present application, R₁, R₂ and R₃ are the same or different and each independently selected from hydrogen, C₁₋₈ alkyl, C₂₋₈ alkenyl, C₂₋₈ alkynyl, C₁₋₈ alkoxy, C₃₋₈ cycloalkyl, C₃₋₈ cycloalkyloxy, 5- to 10-membered heterocyclic groups, C₆₋₁₀ aryl, 5- to 10-membered heteroaryl or 5- to 10-membered heterocyclic groups substituted with one, two or more Ra; where the one, two or more Ra are the same or different and each independently selected from C₆₋₁₀ arylacyl.

According to an embodiment of the present application, R₁, R₂ and R₃ are the same or different and each independently selected from hydrogen, C₁₋₆ alkyl, C₂₋₆ alkenyl, C₂₋₆ alkynyl, C₁₋₆ alkoxy, C₃₋₆ cycloalkyl, C₃₋₆ cycloalkyloxy, 5- to 8-membered heterocyclic groups, C₆₋₈ aryl, 5-to 8-membered heteroaryl or 5- to 8-membered heterocyclic groups substituted with one, two or more Ra; where the one, two or more Ra are the same or different and each independently selected from C₆₋₁₀ arylacyl, for example, benzoyl.

According to an embodiment of the present application, R₁ is selected from hydrogen, methyl, ethyl, isopropyl, isobutyl, t-butyl, cyclopropyl, cyclobutyl, cyclopentyl or cyclohexyl; R₂ is selected from hydrogen, methyl, ethyl, isopropyl, isobutyl, t-butyl, cyclopropyl, cyclobutyl, cyclopentyl or cyclohexyl; and R₃ is selected from methyl, ethyl, isopropyl, t-butyl, isobutyl, methoxy, ethoxy, isopropoxy, t-butoxy, isobutoxy, cyclopropoxy, cyclobutoxy, cyclopentyloxy, cyclohexyloxy or 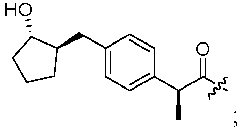 where 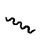 represents a linkage site.

According to an embodiment of the present application, the compound represented by Formula (I) is selected from the following structures:

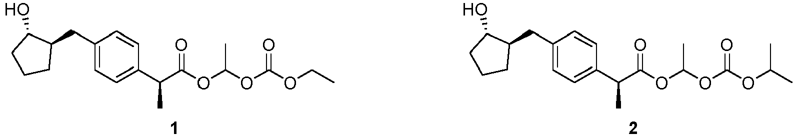

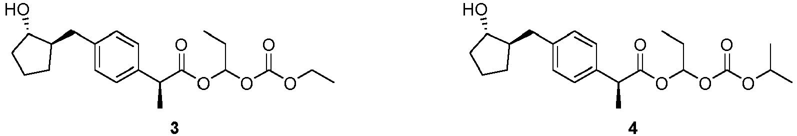

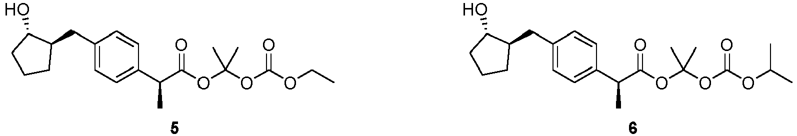

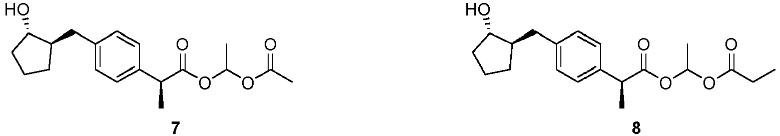

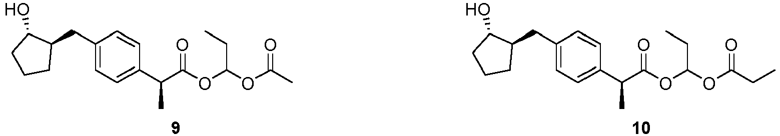

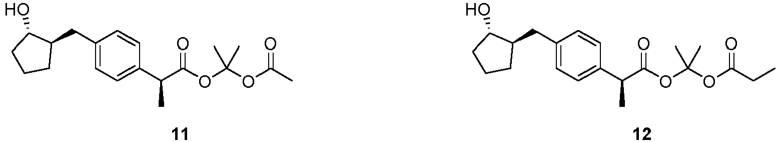

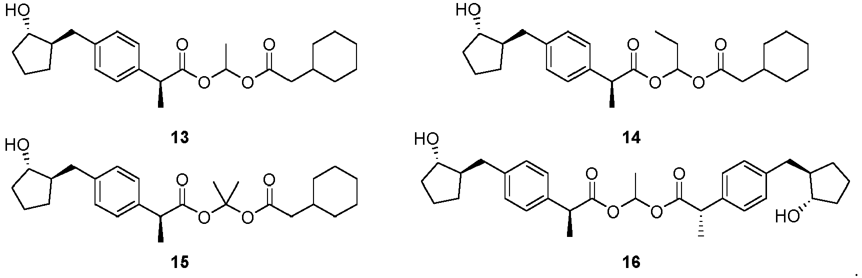

In a second aspect, the present application provides a method for preparing the compound represented by Structural Formula (I) or a racemate, stereoisomer or pharmaceutically acceptable salt or solvate thereof. The method includes: reacting Compound a with Compound b to give the compound represented by Formula (I): 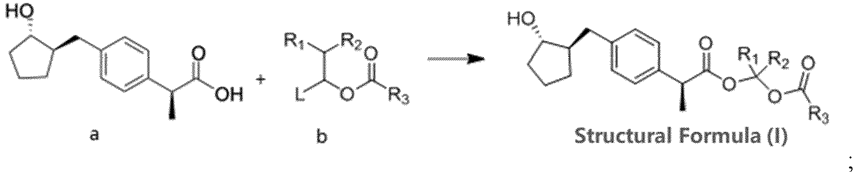
wherein R₁, R₂ and R₃ each independently have the definitions as described above;
L is selected from leaving groups, for example, halogen and hydroxyl; and
Compound a is an active metabolite of loxoprofen, that is, (S)-2-(4-(((1R, 2S)-2-hydroxycyclopentyl)methyl)phenyl)propionic acid.

According to an embodiment of the present application, Compound b is selected from a compound represented by Structural Formula 3 or Structural Formula 4: 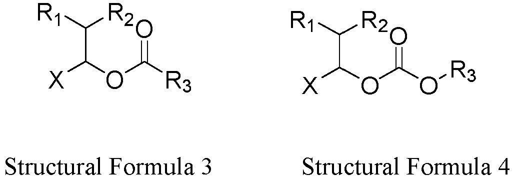
wherein R₁, R₂ and R₃ each independently have the definitions as described above; and
X is selected from chlorine, bromine or iodine.

According to an embodiment of the present application, the preparation method may be performed in the presence of an organic solvent. For example, the organic solvent may be selected from at least one of acetone, dimethylsulfoxide, N,N-dimethylformamide; ethers such as ethyl propyl ether, n-butyl ether, methyl phenyl ether, ethyl phenyl ether, cyclohexyl methyl ether, dimethyl ether, diethyl ether, dimethyl glycol, diphenyl ether, dipropyl ether, diisopropyl ether, di-n-butyl ether, diisobutyl ether, diisopentyl ether, ethylene glycol dimethyl ether, isopropyl ethyl ether, methyl t-butyl ether, tetrahydrofuran, methyl tetrahydrofuran, dioxane, dichlorodiethyl ether and polyethers of ethylene oxide and/or propylene oxide; aliphatic, cycloaliphatic or aromatic hydrocarbons such as pentane, hexane, heptane, octane and nonane; classes that may be substituted with fluorine and chlorine atoms, such as dichloromethane, chloroform, carbon tetrachloride, fluorobenzene, chlorobenzene or dichlorobenzene; cyclohexane, methylcyclohexane, petroleum ether, octane, benzene, toluene, chlorobenzene, bromobenzene, xylene; and esters such as methyl acetate, ethyl acetate, butyl acetate, isobutyl acetate, dimethyl carbonate, dibutyl carbonate or vinyl carbonate.

According to an embodiment of the present application, the preparation method may be performed in the presence of an acid-binding agent such as a base. The base may be an organic base or an inorganic base. The inorganic base may be selected from at least one of a hydride, hydroxide, alkoxide, acetate, fluoride, phosphate, carbonate and bicarbonate of an alkali metal or an alkaline-earth metal, and a preferred base is sodium amino, sodium hydride, lithium diisopropylamino, sodium methoxide, potassium tert-butoxide, sodium hydroxide, potassium hydroxide, sodium acetate, sodium phosphate, potassium phosphate, potassium fluoride, cesium fluoride, sodium carbonate, potassium carbonate, potassium bicarbonate, sodium bicarbonate and cesium carbonate. The organic base may be selected from at least one of tertiary amines, substituted or unsubstituted pyridines and substituted or unsubstituted triethylamine, trimethylamine, N,N-diisopropylethylamine, tri-n-propylamine, tri-n-butylamine, tri-n-hexylamine, tricyclohexylamine, N-methylcyclohexylamine, N-methylpyrrolidine, N-methylpiperidine, N-ethylpiperidine, N,N-dimethylaniline, N-methylmorpholine, pyridine, 2-, 3- or 4-methylpyridine, 2-methyl-5-ethylpyridine, 2,6-dimethylpyridine, 2,4,6-trimethylpyridine, 4-dimethylaminopyridine, quinoline, methylquinoline, N,N,N,N-tetramethyl ethylene diamine, N,N-dimethyl-1,4-diazacyclohexane, N,N-diethyl-1,4-diazacyclohexane, 1,8-bis(dimethylamino)naphthalene, diazabicyclooctane (DABCO), diazabicyclononane (DBN), diazabicycloundecane (DBU), butyl imidazole or methyl imidazole.

According to an embodiment of the present application, the preparation method may be performed in the presence of a catalyst such as a phase transfer catalyst; wherein the catalyst may be selected from tetrabutylammonium bromide (TBAB), tetrabutylammonium chloride (TBAC), tetrabutylammonium iodide (TBAI), potassium iodide, sodium iodide or 18-crown ether-6.

According to an embodiment of the present application, the preparation method is performed at a reaction temperature of -5-80 °C, for example, 0-50 °C. Exemplarily, the reaction temperature is 10 °C, 20 °C, 25 °C, 30 °C or 40 °C.

According to an embodiment of the present application, the preparation method is performed for 0.5-24 h, for example, 1-12 h. Exemplarily, the preparation method is performed for 1 h, 2 h, 3 h, 4 h, 5 h or 6 h.

According to an embodiment of the present application, the compound represented by Structural Formula (I) is a compound represented by Structural Formula 1 or Structural Formula 2:

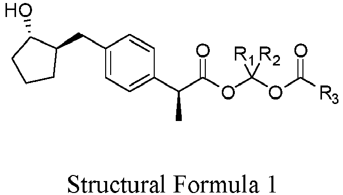

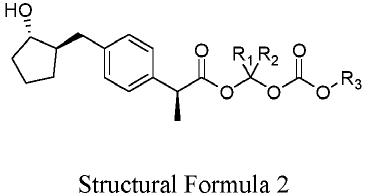

A method for preparing the compound represented by Structural Formula 1, which is provided in the present application, has the following reaction formula: 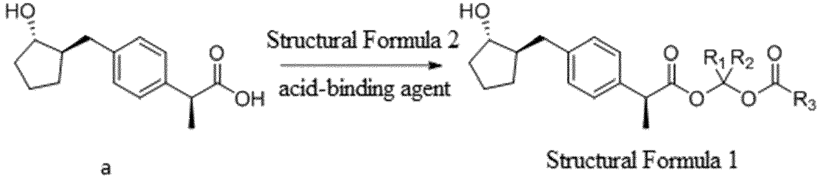 wherein R₁ and R₂ are each independently hydrogen, methyl, ethyl, isopropyl, isobutyl, t-butyl, cyclopropyl, cyclobutyl, cyclopentyl, cyclohexyl or the like; R₁ and R₂ cannot be hydrogen at the same time; and R₃ is methyl, ethyl, isopropyl, t-butyl, isobutyl, cyclopropyl, cyclobutyl, cyclopentyl or cyclohexyl.

The method for preparing the compound represented by Structural Formula 1 is as follows:
at a certain temperature, a certain amount of Compound a and a compound represented by Structural Formula (3), 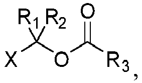 (X is chlorine, bromine or iodine, R₁ and R₂ are each independently hydrogen, methyl, ethyl, isopropyl, isobutyl, t-butyl, cyclopropyl, cyclobutyl, cyclopentyl, cyclohexyl or the like, R₁ and R₂ cannot be hydrogen at the same time, and R₃ is methyl, ethyl, isopropyl, t-butyl, isobutyl, cyclopropyl, cyclobutyl, cyclopentyl, cyclohexyl or the like) are put in a reaction vessel and mixed with an appropriate amount of reaction solvent, an acid-binding agent is slowly added to the reaction vessel, and after addition, the reaction solution is stirred and reacted at a certain temperature for a period of time. When X is Cl, the reaction may be accelerated by a phase transfer catalyst added to the reaction or through heating. During the post-reaction treatment, the reaction solution is extracted, washed, dried, concentrated and subjected to column chromatography to obtain the target compound.

In a preparation process, the reaction temperature is -5-80 °C and the total reaction time is 0.5-24 h; the acid-binding agent used is one or more of inorganic bases including NaOH, KOH, K₂CO₃, KHCO₃, Na₂CO₃ and NaHCO₃ or organic bases including triethylamine, pyridine, DMAP, DIEA and DBU; the reaction solvent is one or more of acetone, dichloromethane, chloroform, carbon tetrachloride, tetrahydrofuran, acetonitrile, DMF, DMAc, ethyl acetate or ether; and the phase transfer catalyst used may be tetrabutylammonium bromide, 18-crown ether-6 or the like. For synthesis methods of the specific compound, see specific examples.

A method for preparing the compound represented by Structural Formula 2, which is provided in the present application, has the following reaction formula: 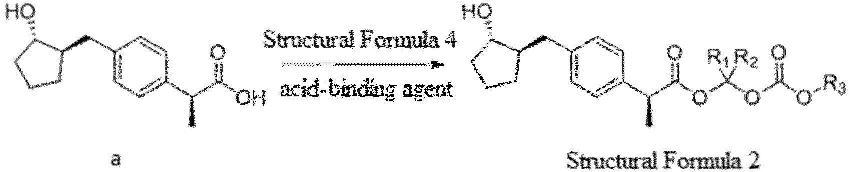 wherein R₁ and R₂ are each independently hydrogen, methyl, ethyl, isopropyl, isobutyl, t-butyl, cyclopropyl, cyclobutyl, cyclopentyl, cyclohexyl or the like; R₁ and R₂ cannot be hydrogen at the same time; and R₃ is methyl, ethyl, isopropyl, t-butyl, isobutyl, cyclopropyl, cyclobutyl, cyclopentyl, cyclohexyl or the like.

The method for preparing the compound represented by Structural Formula 2 is as follows: at a certain temperature, a certain amount of Compound a and a compound represented by Structural Formula (4), 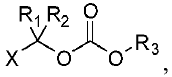 (X is chlorine, bromine or iodine, R₁ and R₂ are each independently hydrogen, methyl, ethyl, isopropyl, isobutyl, t-butyl, cyclopropyl, cyclobutyl, cyclopentyl, cyclohexyl or the like, R₁ and R₂ cannot be hydrogen at the same time, and R₃ is methyl, ethyl, isopropyl, t-butyl, isobutyl, cyclopropyl, cyclobutyl, cyclopentyl, cyclohexyl, 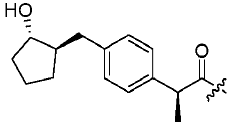 or the like) are put in a reaction vessel and mixed with an appropriate amount of reaction solvent, an acid-binding agent is slowly added to the reaction vessel, and after addition, the reaction solution is stirred and reacted at a certain temperature for a period of time. When X is Cl, the reaction may be accelerated by a phase transfer catalyst added to the reaction or through heating. During the post-reaction treatment, the reaction solution is extracted, washed, dried, concentrated and subjected to column chromatography to obtain the target compound.

In a preparation process, the reaction temperature is -5-80 °C and the total reaction time is 0.5-24 h; the acid-binding agent used is one or more of inorganic bases including NaOH, KOH, K₂CO₃, KHCO₃, Na₂CO₃ and NaHCO₃ or organic bases including triethylamine, pyridine, DMAP, DIEA and DBU; the reaction solvent is one or more of acetone, dichloromethane, chloroform, carbon tetrachloride, tetrahydrofuran, acetonitrile, DMF, DMAc, ethyl acetate or ether; and the phase transfer catalyst used may be tetrabutylammonium bromide, 18-crown ether-6 or the like. For specific synthesis methods of the compound, see specific examples.

According to an embodiment of the present application, when R₂ is hydrogen, a method for preparing a halogenated organic acid ester represented by Structural Formula 3 includes: reacting an aldehyde with organic acyl chloride or organic acyl bromide in the presence of a catalyst.

The reaction formula is as follows: 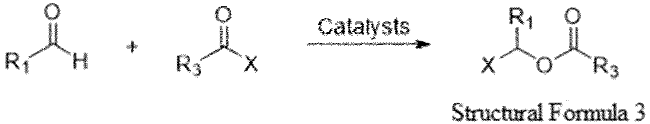 wherein X is chlorine or bromine, R₁ is methyl, ethyl, isopropyl, isobutyl, t-butyl, cyclopropyl, cyclobutyl, cyclopentyl, cyclohexyl or the like; and R₃ is methyl, ethyl, isopropyl, t-butyl, isobutyl, cyclopropyl, cyclobutyl, cyclopentyl, cyclohexyl or the like.

The method for preparing the compound represented by Structural Formula 3 is as follows: at a certain temperature, a certain amount of alkylacyl chloride or alkylacyl bromide 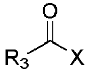 (R₃ is methyl, ethyl, isopropyl, isobutyl, t-butyl, cyclopropyl, cyclobutyl, cyclopentyl, cyclohexyl or the like) and the catalyst (such as ZnCl₂) are put in a reaction vessel, a reaction solvent is added and stirred continuously, the reaction vessel is purged with N₂ three times, then an aldehyde compound 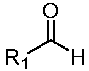 (R₁ is methyl, ethyl, isopropyl, t-butyl, isobutyl, cyclopropyl, cyclobutyl, cyclopentyl, cyclohexyl or the like) is slowly added to the reaction vessel, and after addition, the reaction is adjusted to a certain temperature and carried out for a period of time and then the reaction solution is concentrated for the solvent to be removed, washed and concentrated or distilled to obtain the target compound.

In the preceding preparation process, the reaction temperature is -5-80 °C and the total reaction time is 1-8 h. When 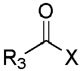 is acyl chloride (X is chlorine), the reaction of 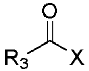 with the aldehyde needs to be heated and carried out for a longer time. The catalyst used is generally ZnCl₂ and the solvent used is one or more of acetone, dichloromethane, chloroform, carbon tetrachloride, tetrahydrofuran, DMF, toluene or ether. For specific synthesis methods of the compound, see specific examples.

According to an embodiment of the present application, when R₂ is hydrogen, a method for preparing a halogenated organic carbonate (X is chlorine or iodine) represented by Structural Formula 4 includes: reacting an organic aldehyde with triphosgene at a low temperature to obtain an intermediate, a chloroalkyl formate and then reacting the chloroalkyl formate with an corresponding organic alcohol to obtain a chlorinated organic carbonate. The chlorinated organic carbonate may be further reacted with NaI to synthesize an iodinated organic carbonate.

The reaction formula is as follows: 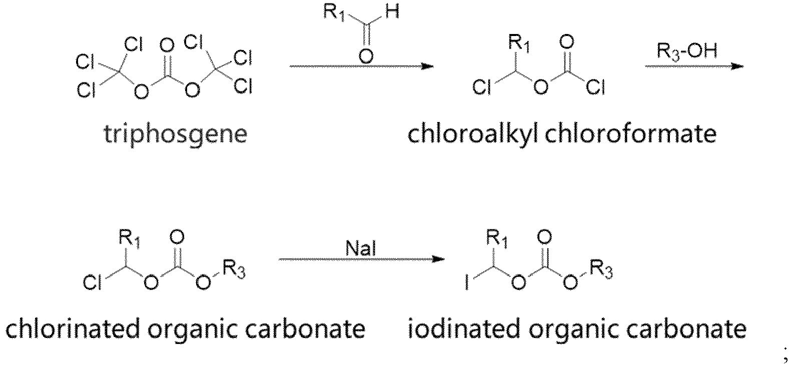 wherein R₁ is methyl, ethyl, isopropyl, isobutyl, t-butyl, cyclopropyl, cyclobutyl, cyclopentyl, cyclohexyl or the like; and R₃ is methyl, ethyl, isopropyl, t-butyl, isobutyl, cyclopropyl, cyclobutyl, cyclopentyl, cyclohexyl or the like.

According to an embodiment of the present application, the method for preparing the chlorinated organic carbonate (X is Cl) represented by Structural Formula 4 is as follows:
step 1: triphosgene, an acid-binding agent and a reaction solvent are added to a reaction vessel placed in a low temperature environment and under N₂ protection, an aldehyde compound 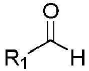 is slowly added dropwise to the reaction vessel, after addition, the reaction is continued for a certain period of time at 0 °C and pumped under reduced pressure (the removed gas is absorbed by lye), and the residual solution is concentrated under reduced pressure at room temperature for the solvent to be removed and distilled to obtain a chloroalkyl chloroformate;
step 2: the chloroalkyl chloroformate obtained in the preceding step and an alkyl alcohol R₃-OH are separately added to a water-free and oxygen-free reaction vessel containing a reaction solvent, placed in an ice bath of 0 °C, and slowly added with an acid-binding agent (such as pyridine), and after addition, the reaction is moved to room temperature and continues to be stirred and react for a period of time; then, the reaction solution is washed, dried and concentrated under reduced pressure to obtain the chlorinated organic carbonate (X is Cl) represented by Structural Formula 4; and
step 3: under N₂ protection, the chlorinated organic carbonate prepared earlier, anhydrous NaI, a phase transfer catalyst, desiccant or the like are placed in a reaction flask and mixed with a solvent, the reaction is heated for a certain period of time, and the reaction is cooled to room temperature, washed with 5%-25% sodium thiosulfate, water and saturated salt solution in sequence, dried and concentrated for the solvent to be removed or further distilled to obtain the iodinated organic carbonate.

In the preceding preparation process, the reaction solvent may be one or more of acetone, dichloromethane, chloroform, carbon tetrachloride or ether; the acid-binding agent may be one or more of pyridine, triethylamine, DIEA, DBU, NaOH, KOH, K₂CO₃, KHCO₃, Na₂CO₃ or NaHCO₃; and the reaction time is generally 0.5-2 h. The final compound obtained may be directly used in the next step without purification. For specific synthesis methods of the compound, see specific examples.

According to an embodiment of the present application, the present application provides a method for preparing an iodinated organic carbonate represented by Structural Formula 4 (X in Structural Formula 4 is iodine). The method includes: reacting a chlorinated organic carbonate prepared earlier with NaI.

The reaction formula is as follows: 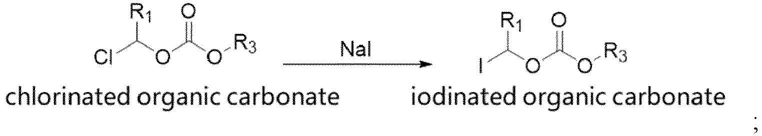 wherein R₁ is methyl, ethyl, isopropyl, isobutyl, t-butyl, cyclopropyl, cyclobutyl, cyclopentyl, cyclohexyl or the like; and R₃ is methyl, ethyl, isopropyl, t-butyl, isobutyl, cyclopropyl, cyclobutyl, cyclopentyl, cyclohexyl or the like.

The preceding method for preparing the iodinated organic carbonate (X in Structural Formula 4 is iodine) is as follows: under N₂ protection, the chlorinated organic carbonate prepared earlier, anhydrous NaI, a phase transfer catalyst, desiccant or the like are placed in a reaction flask and mixed with a solvent, the reaction is heated for a certain period of time, and then the reaction is cooled to room temperature, washed with 5%-25% sodium thiosulfate, water and saturated salt solution in sequence, dried and concentrated for the solvent to be removed or further distilled to obtain the iodinated organic carbonate.

In the preceding preparation process, the phase transfer catalyst used may be 18-crown ether-6, tetrabutylammonium bromide or the like; the desiccant that may be added to the reaction may be CaCl₂, MgSO₄, Na₂SO₄ or the like; the reaction solvent may be acetonitrile, ethyl acetate, DMF, toluene, tetrahydrofuran, DMAc or the like; the reaction temperature is 25-100 °C; and the reaction time is 1-12 h.

In a third aspect, the present application provides an application of the preceding compound represented by Structural Formula (I) or a racemate, stereoisomer or pharmaceutically acceptable salt or solvate thereof to preparation of a nonsteroidal anti-inflammatory drug.

According to an embodiment of the present application, the drug may be used for anti-inflammation and/or analgesia of rheumatoid arthritis, low back pain, migraine, neuralgia, periarthritis of shoulder or osteoarthritis, neck-shoulder-wrist syndromes, analgesia and/or anti-inflammation after surgery, trauma or tooth extraction, and antipyretic and/or analgesia of acute upper respiratory tract inflammation.

The present application further provides a method for anti-inflammation and/or analgesia of rheumatoid arthritis, low back pain, migraine, neuralgia, periarthritis of shoulder or osteoarthritis, neck-shoulder-wrist syndromes, analgesia and/or anti-inflammation after surgery, trauma or tooth extraction, and antipyretic and/or analgesia of acute upper respiratory tract inflammation. The method includes: administering a patient with a prophylactically or therapeutically effective amount of at least one of the compound represented by Structural Formula (I) or the racemate, stereoisomer or pharmaceutically acceptable salt or solvate thereof.

In some embodiments, the patient is a human.

The present application further provides a compound represented by Structural Formula (I), a racemate, stereoisomer or pharmaceutically acceptable salt or solvate thereof or a pharmaceutical composition thereof, which is used for anti-inflammation and/or analgesia of rheumatoid arthritis, low back pain, migraine, neuralgia, periarthritis of shoulder or osteoarthritis, neck-shoulder-wrist syndromes, analgesia and/or anti-inflammation after surgery, trauma or tooth extraction, and antipyretic and/or analgesia of acute upper respiratory tract inflammation.

As a drug, the compound of the present application may be administered in the form of the pharmaceutical composition. Such compositions may be prepared in manners well-known in the field of pharmaceutical preparations and may be administered by a variety of routes, which depends on a need for local or systemic treatment and an area to be treated. Topical administration (for example, transdermal delivery, delivery through skin, eyes and mucous membranes (including intranasal, vaginal and rectal delivery)), pulmonary administration (for example, inhaling or insufflating powder or aerosols, for example, by a nebulizer, intratracheal administration or intranasal administration), oral administration or parenteral administration may be practiced. Parenteral administration includes intravenous, intra-arterial, subcutaneous, intraperitoneal or intramuscular injection or infusion, or intracranial administration such as intrathecal or intraventricular administration. The pharmaceutical composition may be administered parenterally in the form of a single high-dose or may be administered through, for example, a continuous infusion pump. Pharmaceutical compositions and preparations administered topically may include transdermal patches, ointments, lotions, creams, gels, drops, suppositories, spray, liquids, fat emulsion injections and powder. Conventional drug carriers, water, powder or oily substrates, thickening agents and the like may be necessary or needed.

When the pharmaceutical composition of the present application is prepared, an active ingredient is typically mixed with an excipient and diluted by the excipient or loaded into such carriers, for example, capsules, sachets, paper or other containers. When used as a diluent, the excipient may be a solid, semi-solid or liquid substance and used as a vehicle, a carrier or a medium of the active ingredient. Thus, the composition may be in the form of a tablet, a pill, powder, a capsule, an injection, a lozenge, a sachet, a cachet, an elixir, a suspension, an emulsion, a solution, an eye drop, syrup, a gel, an ointment, an aerosol (solid or soluble in a liquid vehicle) or cataplasma; or an ointment, a soft and hard gelatin capsule, a suppository, a sterile injection solution and sterile packaged powder containing, for example, up to 10% by weight of active compound.

Some examples of suitable excipients include lactose, glucose, sucrose, sorbitol, mannitol, starch, arabic gum, calcium phosphate, alginate, tragacanth, gelatin, calcium silicate, microcrystalline cellulose, polyvinylpyrrolidone, cellulose, water, syrup and methylcellulose. Preparations may also contain lubricants such as talc, magnesium stearate and mineral oil; wetting agent; emulsifiers and suspending agents; preservatives such as methyl benzoate and hydroxypropyl benzoate; and sweeteners and correctives. The composition of the present application may be formulated by methods known in the art for an immediate, extended or delayed release of the active ingredient after administered to a patient.

The composition may be formulated in a unit dosage form, each dosage containing about 5-1000 mg, more typically about 100-500 mg of active ingredient. The term "unit dosage form" refers to a physically separated single dosage unit suitable for use in human patients and other mammal, each unit containing a predetermined amount of active substance, which is calculated to produce desired efficacy in combination with a suitable drug excipient.

The active compound may have a very large effective dosage range and is generally administered at a pharmaceutically effective dosage. However, it is to be understood that an amount of the compound actually administered is generally determined by a physician according to related factors which include a condition to be treated, a selected route of administration, an actual compound administered, an age, weight and response of an individual patient, severity of a symptom of the patient and the like.

For preparation of solid compositions such as tablets, the main active ingredient is mixed with a drug excipient to form a solid preformulation composition containing a homogeneous mixture of the compound of the present application. A homogeneous preformulation composition refers to that the active ingredient is generally uniformly distributed throughout the composition so that the composition can easily be divided into equally effective unit dosage forms such as tablets, pills and capsules. The solid preformulation is then divided into the above types of unit dosage form containing, for example, about 0.1-1000 mg of the active ingredient of the present application.

The tablets or pills of the present application may be coated or compounded to obtain a dosage form that provides long-lasting efficacy. For example, the tablet or the pill contains both inner and outer dosage components, the outer dosage component being a coating of the inner dosage component. The inner and outer dosage components may be separated by an enteric layer for preventing disintegration in the stomach so that the inner component is intact through the duodenum or its release is delayed. A variety of substances may be used as such enteric layers or coating agents. Such substances include a variety of polymer acids and mixtures of polymer acids with substances such as shellac, cetyl alcohol and cellulose acetate.

A liquid form into which the compound and composition of the present application may be incorporated for oral administration or injection includes an aqueous solution or suitably tasted syrup, water or oil suspension; an emulsion prepared from edible oils such as cottonseed oil, sesame oil, medium chain oil, coconut oil or peanut oil; and an elixir and similar pharmaceutical vehicles.

Compositions inhaled or insufflated include solutions and suspensions soluble in pharmaceutically acceptable water or organic solvents or mixtures thereof and powder. Liquid or solid compositions may contain suitable pharmaceutically acceptable excipients as described above. In some embodiments, the composition is administered by oral or nasal routes to achieve a local or systemic effect. The composition may be atomized by using an inert gas. An atomized solution may be inhaled directly through an atomizing device or the atomizing device may be connected to a protective face mask or an intermittent positive pressure respirator. Solutions, suspensions or powder of the composition may be administered through oral administration or through nasal administration by a device that delivers preparations in an appropriate manner.

The amount of the compound or composition administered to the patient is variable and depends on a drug administered, a purpose of administration such as prevention or treatment, a state of the patient, a route of administration and the like. When used for treatment, the composition may be administered to a patient suffering from a disease in an amount sufficient to cure or at least partially inhibit the disease and symptoms of complications thereof. The effective dosage should depend on a state of the disease to be treated and a decision of an attending clinician, where the decision depends on factors such as the severity of the disease and the age, weight and general conditions of the patient.

The composition administered to the patient may be in the preceding form of the pharmaceutical composition. These compositions may be sterilized by conventional sterilization techniques or through filtration. The aqueous solution may be packaged as it is or lyophilized. A lyophilized preparation is mixed with a sterile aqueous carrier before administered. The preparation of the compound generally has a pH of 3-11, more preferably 5-9, most preferably 7-8. It is to be understood that the use of some excipients, carriers or stabilizers described above results in the formation of drug salts.

A therapeutic dosage of the compound of the present application may depend on, for example, a specific use of treatment, the route of administration, the health and state of the patient and the decision of a prescribing physician. A proportion or concentration of the compound of the present application in the pharmaceutical composition may be variable and depends on multiple factors including the dosage, chemical properties (such as hydrophobicity) and the route of administration. For example, the compound of the present application may be provided by a physiological buffer aqueous solution containing about 0.1-10 %w/v of the compound for parenteral administration. A certain typical dosage range is about 1 µg/kg to about 1 g/kg body weight per day. In some embodiments, the dosage range is about 0.01 mg/kg to about 100 mg/kg body weight per day. The dosage is likely to depend on such variables as the type and development of a disease or condition, the general health state of a particular patient, the relative biological potency of the selected compound, an excipient preparation and the route of administration. The effective dosage may be extrapolated from a dosage-response curve derived from an in vitro or animal model test system.

### BRIEF DESCRIPTION OF DRAWINGS

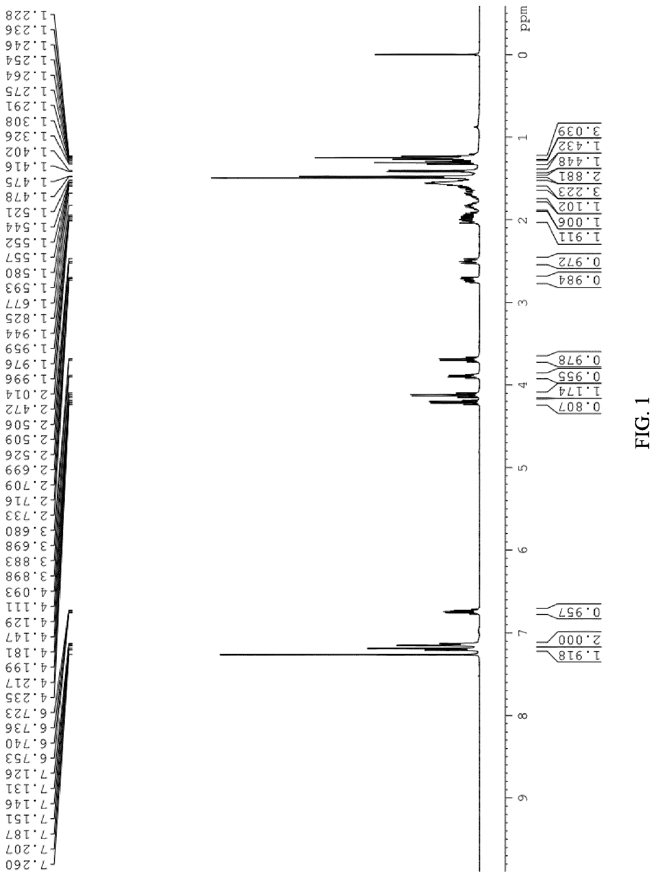

FIG. 1 shows an ¹H NMR spectrum of Compound 1 of the present application.

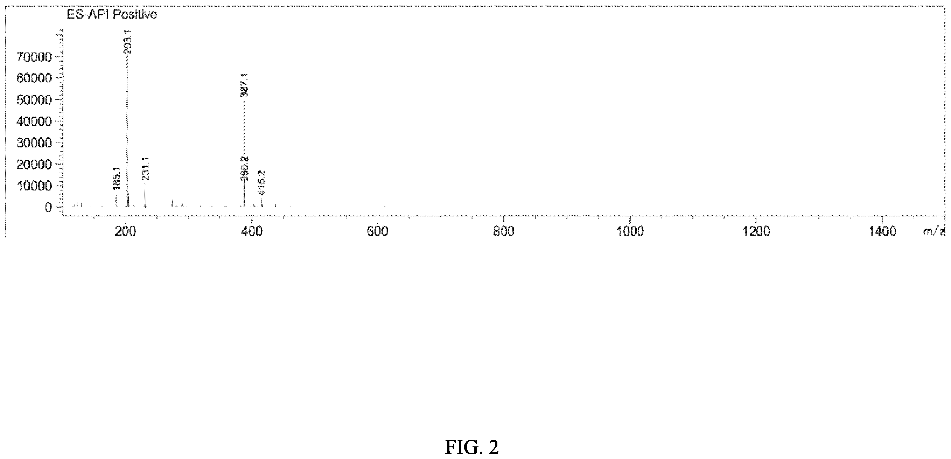

FIG. 2 shows a mass spectrum of Compound 1 of the present application.

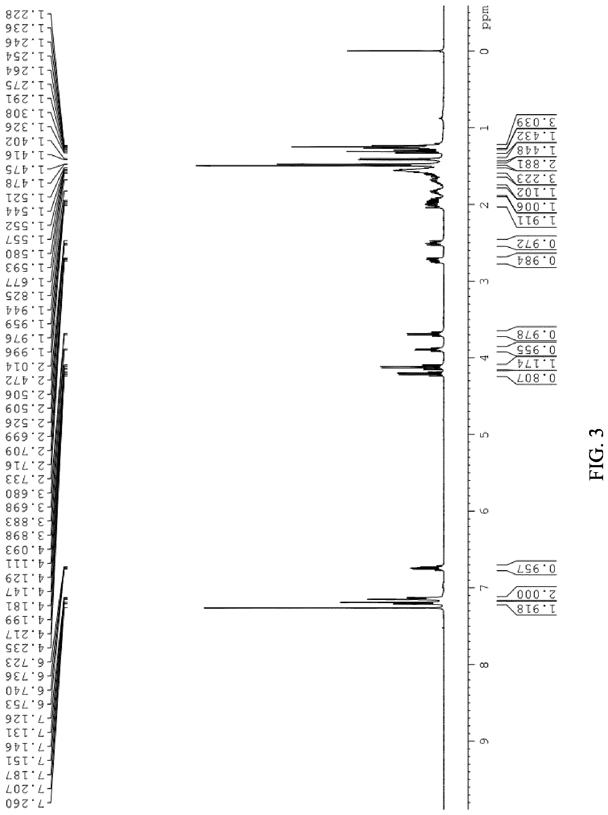

FIG. 3 shows an ¹H NMR spectrum of Compound 7 of the present application.

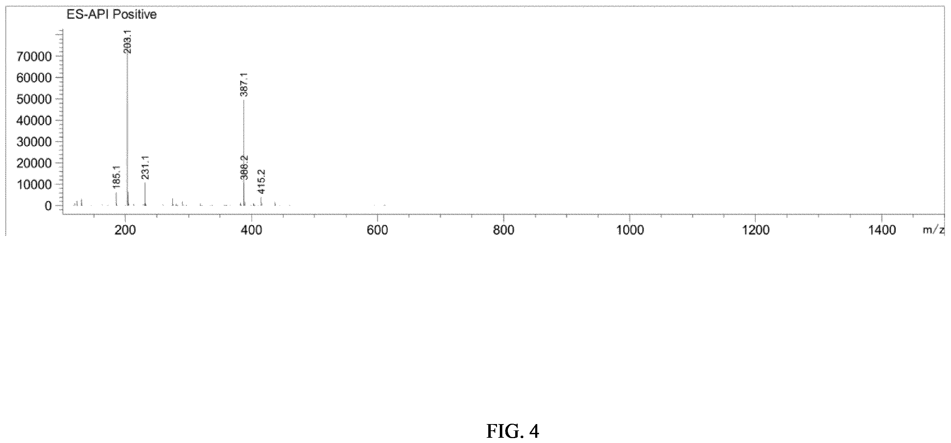

FIG. 4 shows a mass spectrum of Compound 7 of the present application.
FIG. 5 shows degradation of Compounds 1 and 7 of the present application in human plasma.
FIG. 6 shows generation of Compounds 1 and 7 of the present application in human plasma.

### DETAILED DESCRIPTION

Technical solutions of the present application are described below in more detail in conjunction with specific examples. It is to be understood that the following examples are merely intended to exemplarily describe and explain the present application and not to be construed as limiting the scope of the present application. Any techniques achieved based on the preceding content of the present application are within the scope of the present application.

Unless otherwise specified, raw materials and reagents used in the following examples are all commercially available products or can be prepared by known methods.

The present application provides an arylpropionic acid derivative. The compound has a structure represented by Structural Formula 1 or Structural Formula 2: 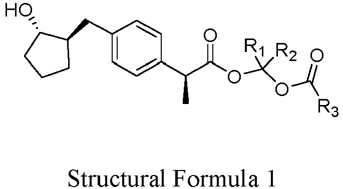 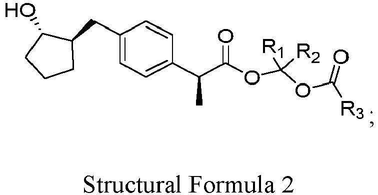 wherein R₁ and R₂ are each independently hydrogen, methyl, ethyl, isopropyl, isobutyl, t-butyl, cyclopropyl, cyclobutyl, cyclopentyl, cyclohexyl or the like; R₁ and R₂ cannot be hydrogen at the same time; and R₃ is methyl, ethyl, isopropyl, t-butyl, isobutyl, cyclopropyl, cyclobutyl, cyclopentyl, cyclohexyl, 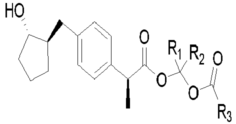 or the like.

General synthesis formula I (for Compounds N7 to N12 with Structural Formula 1) 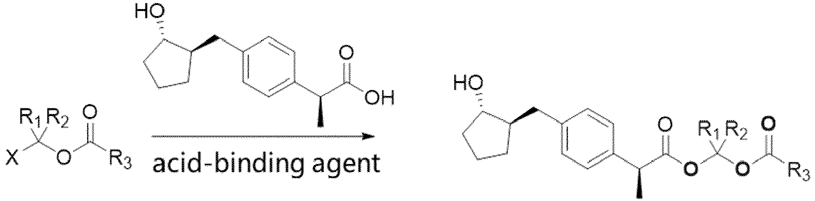 wherein X is chlorine, bromine or iodine; R₁ and R₂ are each independently hydrogen, methyl, ethyl, isopropyl, isobutyl, t-butyl, cyclopropyl, cyclobutyl, cyclopentyl, cyclohexyl or the like; R₁ and R₂ cannot be hydrogen at the same time; and R₃ is methyl, ethyl, isopropyl, t-butyl, isobutyl, cyclopropyl, cyclobutyl, cyclopentyl, cyclohexyl, 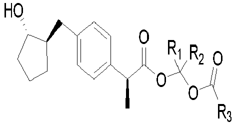 or the like.

### General synthesis method

Synthesis of an iodinated organic acid ester: under N₂ protection, a chlorinated organic carbonate purchased or prepared earlier, anhydrous NaI, a phase transfer catalyst and a desiccant are placed in a reaction flask, mixed with a solvent and reacted at a certain temperature for a certain period of time, and then the reaction is cooled to room temperature, washed with 5%-25% sodium thiosulfate, water and saturated salt solution in sequence, dried and concentrated for the solvent to be removed or further distilled to obtain the iodinated organic acid ester.

Synthesis of a related compound with Structural Formula (1): a certain amount of Compound a (1.0 eq) and an acid-binding agent (1.0 eq) are put in a reaction vessel and mixed with an appropriate amount of reaction solvent, a compound represented by Structural Formula (3), 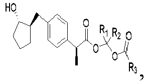 (X is chlorine, bromine or iodine, R₁ and R₂ are each independently hydrogen, methyl, ethyl, isopropyl, isobutyl, t-butyl, cyclopropyl, cyclobutyl, cyclopentyl, cyclohexyl or the like, R₁ and R₂ cannot be hydrogen at the same time, and R₃ is methyl, ethyl, isopropyl, t-butyl, isobutyl, cyclopropyl, cyclobutyl, cyclopentyl, cyclohexyl or the like) is slowly added to the reaction vessel, and after addition, the reaction solution is stirred and reacted at a certain temperature for a period of time. When X is Cl, the reaction may be accelerated by a phase transfer catalyst added to the reaction or through heating. During the post-reaction treatment, the reaction solution is extracted, washed, dried, concentrated and subjected to column chromatography to obtain the target compound.

### General synthesis formula II (for Compounds N1 to N6 and N13 to N15 with Structural Formula 2)

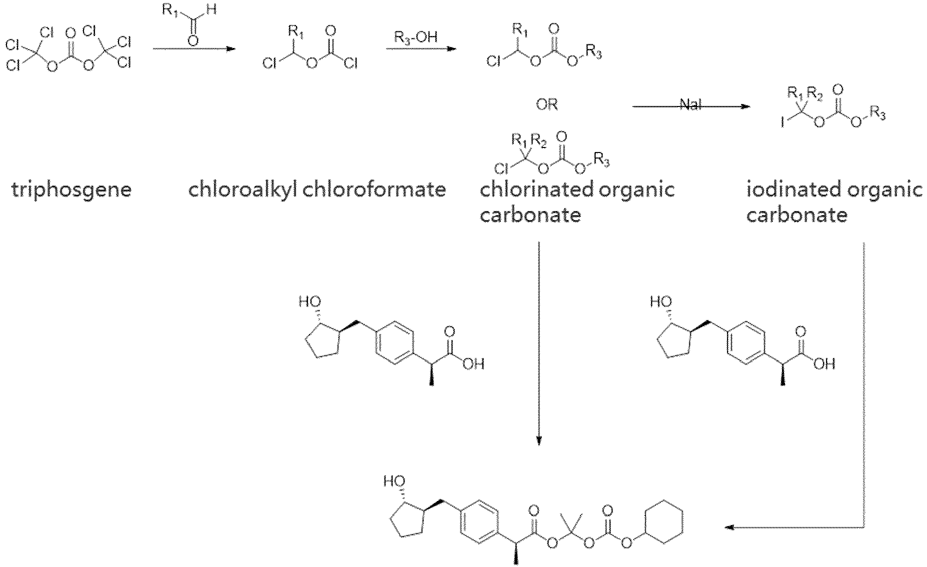

### General synthesis method

When R₂ is hydrogen, a chlorinated organic carbonate has the following general synthesis method:
Synthesis of a chloroalkyl chloroformate: triphosgene, an acid-binding agent and a reaction solvent are added to a reaction vessel placed in a low temperature environment and under N₂ protection, an aldehyde compound 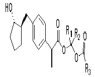 is slowly added dropwise to the reaction vessel, after addition, the reaction is continued for a certain period of time at 0 °C and then pumped under reduced pressure (the removed gas is absorbed by lye), and the residual solution is concentrated under reduced pressure at room temperature for the solvent to be removed and then distilled to obtain the chloroalkyl chloroformate.

Synthesis of the chlorinated organic carbonate: the chloroalkyl chloroformate obtained in the preceding step and an alkyl alcohol 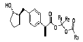 are separately added to a water-free and oxygen-free reaction vessel containing a reaction solvent, placed in an ice bath of 0 °C and slowly added with an acid-binding agent (such as pyridine), and after addition, the reaction is moved to room temperature, stirred and reacted for a certain period of time. Then, the reaction solution is washed, dried and concentrated under reduced pressure to obtain the chlorinated organic carbonate with Structural Formula (4) (X is Cl).

For any R₁ and R₂ groups, the compound with Structural Formula 2 has the following general synthesis method:
Synthesis of an iodinated organic carbonate: under N₂ protection, a chlorinated organic carbonate purchased or prepared earlier, anhydrous NaI and a phase transfer catalyst or desiccant are placed in a reaction flask and mixed with a solvent, the reaction is heated and reacted for a certain period of time, and then the reaction is cooled to room temperature, washed with 5%-25% sodium thiosulfate, water and saturated salt solution in sequence, dried and concentrated for the solvent to be removed or further distilled to obtain the iodinated organic carbonate.

Synthesis of the compound represented by Structural Formula 2: at a certain temperature, a certain amount of ibuprofen and an acid-binding agent are put in a reaction vessel and mixed with an appropriate amount of reaction solvent, a compound represented by Structural Formula 4, 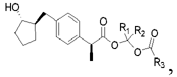 (X is chlorine, bromine or iodine, R₁ and R₂ are each independently hydrogen, methyl, ethyl, isopropyl, isobutyl, t-butyl, cyclopropyl, cyclobutyl, cyclopentyl, cyclohexyl or the like, R₁ and R₂ cannot be hydrogen at the same time, and R₃ is methyl, ethyl, isopropyl, t-butyl, isobutyl, cyclopropyl, cyclobutyl, cyclopentyl, cyclohexyl or the like) is slowly added to the reaction vessel, and after addition, the reaction solution is stirred and reacted at a certain temperature for a period of time. When X is Cl, the reaction may be accelerated by NaI/KI or a phase transfer catalyst added to the reaction or through heating. During the post-reaction treatment, the reaction solution is extracted, washed, dried, concentrated and subjected to column chromatography to obtain the target compound.

### Example 1

### Compound 1 was synthesized according to the following reaction formula:

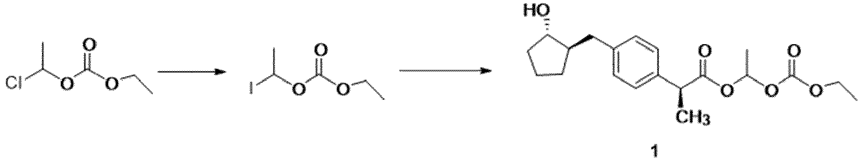

**Synthesis of 1-iodoethyl ethyl carbonate:** 1-chloroethyl ethyl carbonate (2.434 g, 16.01 mmol) was weighed into a dry 100 mL two-necked reaction flask, anhydrous NaI (2.14 g, 14.25 mmol), TBAB (51.6 mg, 0.16 mmol), anhydrous CaCl₂ (649 mg, 5.76 mmol) and ethyl acetate (10 mL) were added, and the system was heated to 80 °C and refluxed for 3 h. The reaction flask was added with water and shaken, layers were separated, and the EA layer was washed with saturated salt solution, dried over anhydrous sodium sulfate and concentrated under reduced pressure to obtain 1-iodoethyl ethyl carbonate as a brown oil, which was directly used in the next step without purification.

**Synthesis of Compound 1:** at room temperature, Compound a (0.717 g, 2.89 mmol) was weighed and added to a dry 50 mL single-necked reaction flask, added with 10 mL of acetone and stirred to be dissolved. Then, 1-iodoethyl ethyl carbonate (2.116 g, 8.67 mmol) was weighed and added to the above reaction flask and DBU (0.443 g, 2.91 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA = 4:1) to obtain the target product (0.768 g, 2.11 mmol) with a yield of 73.0%.

¹H NMR (500 MHz, *d6*-DMSO) δ 7.19(d, *J*= 8.0 Hz, 2H), 7.12(d, *J*= 8.0 Hz, 2H), 6.78-6.82(m, 1H), 4.25(s, 1H), 4.15-4.24(m, 2H), 3.59-3.69(m, 2H), 2.78(dd, *J*= 13.5, 5.4 Hz, 1H), 2.32(dd, *J*= 13.4, 9.4 Hz, 1H), 1.75-1.92(m, 2H), 1.51-1.55(m, 3H), 1.39-1.70(m, 4H), 1.35(d, 3H, *J* = 7.1 Hz), 1.27-1.33(m, 3H), 1.12-1.13(m, 1H).

### Example 2

### Compound 2 was synthesized according to the following reaction formula:

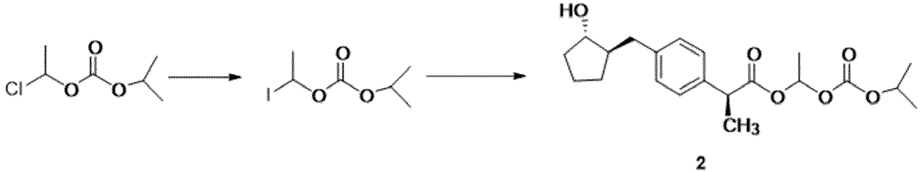

**Synthesis of 1-iodoethyl isopropyl carbonate:** 1-chloroethyl isopropyl carbonate (2.600 g, 15.66 mmol) was weighed into a dry 100 mL two-necked reaction flask, anhydrous NaI (2.09 g, 13.94 mmol), TBAB (50.5 mg, 0.157 mmol), anhydrous CaCl₂ (625.78 mg, 5.64 mmol) and ethyl acetate (12 mL) were added, and the system was heated to 80 °C and refluxed for 3 h. The reaction flask was added with water and shaken, layers were separated, and the EA layer was washed with saturated salt solution, dried over anhydrous sodium sulfate and concentrated under reduced pressure to obtain 1-iodoethyl isopropyl carbonate as a brown oil, which was directly used in the next step without purification.

**Synthesis of Compound 2:** at room temperature, Compound a (1.980 g, 7.98 mmol) was weighed and added to a dry 50 mL single-necked reaction flask, added with 10 mL of acetone and stirred to be dissolved. Then, 1-iodoethyl isopropyl carbonate (3.111 g, 12.06 mmol) was weighed and added to the above reaction flask and DBU (1.200 g, 7.88 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA = 4:1) to obtain the target product (2.016 g, 5.33 mmol) with a yield of 66.8%.

¹H NMR (500 MHz, *d6*-DMSO) *δ* 7.19(d, *J*= 8.0 Hz, 2H), 7.12(d, *J*= 8.0 Hz, 2H), 6.78-6.82(m, 1H), 4.69-4.82(m, 1H), 4.25(s, 1H), 3.59-3.69(m, 2H), 2.78(dd, *J*= 13.5, 5.4 Hz, 1H), 2.32(dd, *J*= 13.4, 9.4 Hz, 1H), 1.75-1.92(m, 2H), 1.51-1.55(m, 3H), 1.39-1.70(m, 4H), 1.35(d, 3H, *J* = 7.1 Hz), 1.26-1.33(m, 6H), 1.12-1.13(m, 1H).

### Example 3

### Compound 3 was synthesized according to the following reaction formula:

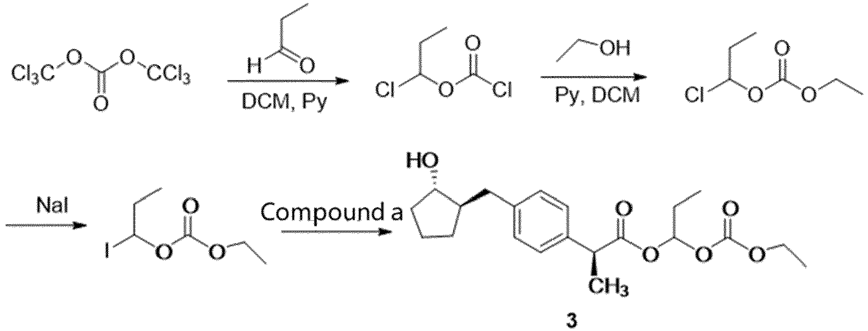

**Synthesis of 1-chloropropyl chloroformate:** triphosgene (10.03 g, 33.70 mmol) was weighed into a 100 mL three-necked reaction flask and added with 15 mL of anhydrous dichloromethane, and the reaction flask was purged with Ar three times, transferred to a cold trap of -20 °C and stirred continuously. Pyridine (0.540 g, 6.83 mmol) was diluted with 5 mL of dichloromethane and then added to the reaction flask. N-propionaldehyde (4.602 g, 79.20 mmol) was weighed and slowly added dropwise to the reaction flask. After dropwise addition, the temperature of the cold trap was set to -20 °C and the reaction was continued for 20 h. The reaction flask was pumped for 5 min using a water pump connected with a reaction flask containing an aqueous solution of KOH, and then transferred out of the cold trap. The solution was concentrated under reduced pressure for DCM to be removed and then distilled to obtain 1-chloropropyl chloroformate (3.91 g) as a colorless to pale yellow oil with a yield of 73.2%.

**Synthesis of 1-chloropropyl ethyl carbonate:** 1-chloropropyl chloroformate (1.011 g, 6.38 mmol) was weighed into a dry two-necked reaction flask, added with 10 mL of anhydrous DCM and stirred continuously. Ethanol (0.440g, 9.55 mmol) was weighed and added to the above reaction flask. The reaction flask was transferred to an ice-water bath and stirred continuously. Pyridine (0.631 g, 7.96 mmol) was weighed and slowly added dropwise to the above reaction flask with a white solid appearing. After dropwise addition, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution and dried over anhydrous sodium sulfate. The resulting product was concentrated under reduced pressure to obtain 1-chloropropyl chloroformate (0.766 g) as a colorless oil, which was directly used in the next step without purification.

**Synthesis of 1-iodopropyl ethyl carbonate:** 1-chloropropyl ethyl carbonate (0.766 g, 4.61 mmol) was weighed into a dry 100 mL two-necked reaction flask, anhydrous NaI (614.5 mg, 4.1 mmol), TBAB (14.86 mg, 0.046 mmol), anhydrous CaCl₂ (184 mg, 1.66 mmol) and ethyl acetate (7 mL) were added, and the system was heated to 80 °C and refluxed for 3 h. The reaction flask was added with water and shaken, layers were separated, and the EA layer was washed with saturated salt solution, dried over anhydrous sodium sulfate and concentrated under reduced pressure to obtain 1-iodopropyl ethyl carbonate (0.757 g) as a brown oil, which was directly used in the next step without purification.

**Synthesis of Compound 3:** at room temperature, Compound a (0.757g, 3.05 mmol) was weighed and added to a dry 50 mL single-necked reaction flask, added with 10 mL of acetone and stirred to be dissolved. Then, 1-iodopropyl ethyl carbonate (1.163 g, 4.51 mmol) was weighed and added to the above reaction flask and DBU (0.472 g, 3.10 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA = 4:1) to obtain the target product (0.840 g, 2.22 mmol) with a yield of 72.8%.

¹H NMR (500 MHz, *d6*-DMSO) *δ* 7.19(d, *J*= 8.0 Hz, 2H), 7.12(d, *J*= 8.0 Hz, 2H), 6.78-6.82(m, 1H), 4.25(s, 1H), 4.15-4.25(m, 2H), 3.59-3.69(m, 2H), 2.78(dd, *J*= 13.5, 5.4 Hz, 1H), 2.32(dd, *J*= 13.4, 9.4 Hz, 1H), 1.82-1.91(m, 2H), 1.75-1.92(m, 2H), 1.39-1.70(m, 4H), 1.35(d, 3H, *J* = 7.1 Hz), 1.26-1.33(m, 3H), 1.12-1.13(m, 1H), 0.96-1.02(m, 3H).

### Example 4

### Compound 4 was synthesized according to the following reaction formula:

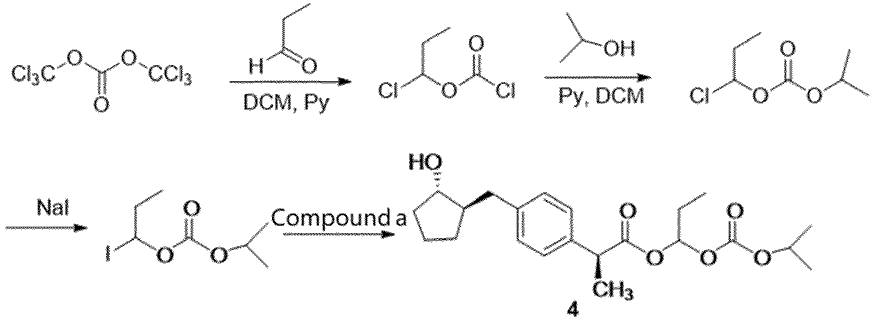

**Synthesis of 1-chloropropyl chloroformate:** triphosgene (10.030 g, 33.70 mmol) was weighed into a 100 mL three-necked reaction flask and added with 15 mL of anhydrous dichloromethane, and the reaction flask was purged with Ar three times, transferred to a cold trap of -20 °C and stirred continuously. Pyridine (0.540 g, 6.83 mmol) was diluted with 5 mL of dichloromethane and then added to the reaction flask. N-propionaldehyde (4.602 g, 79.20 mmol) was weighed and slowly added dropwise to the reaction flask. After dropwise addition, the temperature of the cold trap was set to -2 °C and the reaction was continued for 20 h. The reaction flask was pumped for 5 min using a water pump connected with a reaction flask containing an aqueous solution of KOH, and then transferred out of the cold trap. The solution was concentrated under reduced pressure for DCM to be removed and then distilled to obtain 1-chloropropyl chloroformate (3.91 g) as a colorless to pale yellow oil with a yield of 73.2%.

**Synthesis of 1-chloropropyl isopropyl carbonate:** 1-chloropropyl chloroformate (1.034 g, 6.63 mmol) was weighed into a dry two-necked reaction flask, added with 10 mL of anhydrous DCM, and stirred continuously. Isopropanol (0.598 g, 9.95 mmol) was weighed and added to the above reaction flask. The reaction flask was transferred to an ice-water bath and stirred continuously. Pyridine (0.629 g, 7.96 mmol) was weighed and slowly added dropwise to the above reaction flask with a white solid appearing. After dropwise addition, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution and dried over anhydrous sodium sulfate. The resulting product was concentrated under reduced pressure to obtain 1-chloropropyl isopropyl carbonate (0.822 g) as a colorless oil, which was directly used in the next step without purification.

**Synthesis of 1-iodopropyl isopropyl carbonate:** 1-chloropropyl isopropyl carbonate (0.822 g, 4.57 mmol) was weighed into a dry 100 mL two-necked reaction flask, anhydrous NaI (610 mg, 4.07 mmol), TBAB (14.82 mg, 0.046 mmol), anhydrous CaCl₂ (183 mg, 1.65 mmol) and ethyl acetate (8 mL) were added, and the system was heated to 80 °C and refluxed for 3 h. The reaction flask was added with water and shaken, layers were separated, and the EA layer was washed with saturated salt solution, dried over anhydrous sodium sulfate and concentrated under reduced pressure to obtain 1-iodopropyl isopropyl carbonate (1.055 g) as a brown oil, which was directly used in the next step without purification.

**Synthesis of Compound 4:** at room temperature, Compound a (0.648 g, 2.61 mmol) was weighed and added to a dry 50 mL single-necked reaction flask, added with 10 mL of acetone and stirred to be dissolved. Then, 1-iodopropyl isopropyl carbonate (1.055 g, 3.88 mmol) was weighed and added to the above reaction flask and DBU (0.388 g, 2.55 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA = 4:1) to obtain the target product (0.698 g, 1.78 mmol) with a yield of 68.2%.

¹H NMR (500 MHz, *d6*-DMSO) *δ* 7.19(d, *J*= 8.0 Hz, 2H), 7.12(d, *J* = 8.0 Hz, 2H), 6.78-6.82(m, 1H), 4.69-4.82(m, 1H), 4.25(s, 1H), , 3.59-3.69(m, 2H), 2.78(dd, *J* = 13.5, 5.4 Hz, 1H), 2.32(dd, *J*= 13.4, 9.4 Hz, 1H), 1.82-1.91(m, 2H), 1.75-1.92(m, 2H), 1.39-1.70(m, 4H), 1.35(d, 3H, *J* = 7.1 Hz), 1.26-1.33(m, 6H), 1.12-1.13(m, 1H), 0.96-1.02(m, 3H).

### Example 5

### Compound 5 was synthesized according to the following reaction formula:

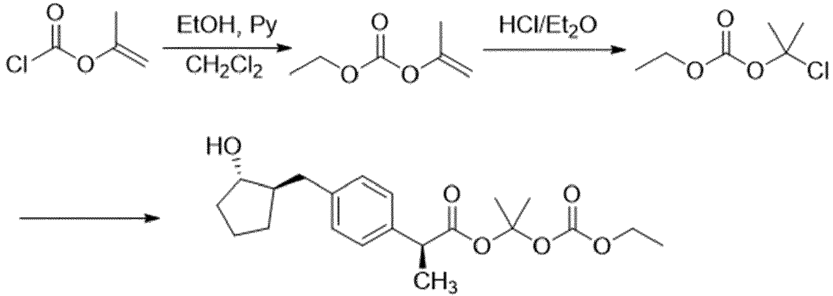

**Synthesis of 1-chloro-1-methyl-ethyl ethyl carbonate:** 2-propenyl chloroformate (1.010 g, 8.30 mmol) was weighed into a single-necked reaction flask, added with 20 mL of anhydrous dichloromethane and stirred continuously. Ethanol (0.382 g, 8.30 mmol) was weighed and added to the above reaction flask and the reaction flask was transferred to an ice bath and stirred. Pyridine (0.691 g, 8.72 mmol) diluted with 5 mL of dry CH₂Cl₂ was slowly added dropwise and then the system was reacted at room temperature for 2 h and poured into 20 mL of ice water. Organic phases were dried over anhydrous sodium sulfate and filtered. The filtrate was evaporated to dryness to obtain an oily liquid. The liquid was added with 10 mL of ether. Then, 50 mL of 4N hydrochloric acid/ether was slowly added dropwise. After addition, the system was stirred overnight at room temperature and concentrated under reduced pressure to obtain 1-chloro-1-methyl-ethyl ethyl carbonate (0.883 g) as a colorless liquid, which was directly used in the next step without purification.

**Synthesis of Compound 5:** at room temperature, Compound a (0.878 g, 3.54 mmol) was weighed and added to a dry 50 mL single-necked reaction flask, added with 10 mL of acetone and stirred to be dissolved. Then, 1-chloro-1-methyl-ethyl ethyl carbonate (0.883 g, 5.32 mmol) was weighed and added to the above reaction flask and DBU (0.542 g, 3.56 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA = 4:1) to obtain the target product (0.753 g, 1.99 mmol) with a yield of 56.2%.

¹H NMR (500 MHz, *d6*-DMSO) *δ* 7.19(d, *J=* 8.0 Hz, 2H), 7.12(d, *J=* 8.0 Hz, 2H), 4.25(s, 1H), 4.15-4.24(m, 2H), 3.59-3.69(m, 2H), 2.78(dd, *J*= 13.5, 5.4 Hz, 1H), 2.32(dd, *J*= 13.4, 9.4 Hz, 1H), 1.75-1.92(m, 2H), 1.61-1.65(m, 6H), 1.39-1.70(m, 4H), 1.35(d, 3H, *J* = 7.1 Hz), 1.27-1.33(m, 3H), 1.12-1.13(m, 1H).

### Example 6

### Compound 6 was synthesized according to the following reaction formula:

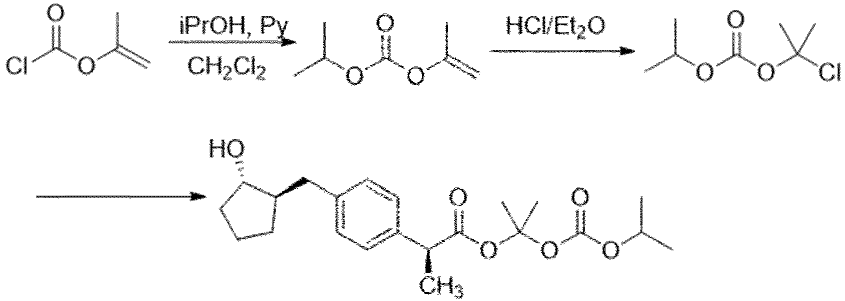

**Synthesis of 1-chloro-1-methyl-ethyl isopropyl carbonate:** 2-propenyl chloroformate (1.010 g, 8.30 mmol) was weighed into a single-necked reaction flask, added with 20 mL of anhydrous dichloromethane and stirred continuously. Isopropanol (0.511 g, 8.50 mmol) was weighed and added to the above reaction flask and the reaction flask was transferred to an ice bath and stirred. Pyridine (0.691 g, 8.72 mmol) diluted with 5 mL of dry CH₂Cl₂ was slowly added dropwise and then the system was reacted at room temperature for 2 h and poured into 20 mL of ice water. Organic phases were dried over anhydrous sodium sulfate and filtered. The filtrate was evaporated to dryness to obtain an oily liquid. The liquid was added with 10 mL of ether. Then, 50 mL of 4N hydrochloric acid/ether was slowly added dropwise. After addition, the system was stirred overnight at room temperature and concentrated under reduced pressure to obtain 1-chloro-1-methyl-ethyl isopropyl carbonate as a colorless liquid, which was directly used in the next step without purification.

**Synthesis of Compound 6:** at room temperature, Compound a (0.797 g, 3.21 mmol) was weighed and added to a dry 50 mL single-necked reaction flask, added with 10 mL of acetone and stirred to be dissolved. Then, 1-chloro-1-methyl-ethyl isopropyl carbonate (0.879 g, 4.88 mmol) was weighed and added to the above reaction flask and DBU (0.483 g, 3.17 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA = 4:1) to obtain the target product (0.698 g, 1.78 mmol) with a yield of 55.5%.

¹H NMR (500MHz, *d6*-DMSO) *δ* 7.19(d, *J*= 8.0 Hz, 2H), 7.12(d, *J*= 8.0 Hz, 2H), 4.69-4.82(m, 1H), 4.25(s, 1H), , 3.59-3.69(m, 2H), 2.78(dd, *J*= 13.5, 5.4 Hz, 1H), 2.32(dd, *J*= 13.4, 9.4 Hz, 1H), 1.75-1.92(m, 2H), 1.61-1.65(m, 6H), 1.39-1.70(m, 4H), 1.35(d, 3H, *J* = 7.1 Hz), 1.26-1.33(m, 6H), 1.12-1.13(m, 1H).

### Example 7

### Compound 7 was synthesized according to the following reaction formula:

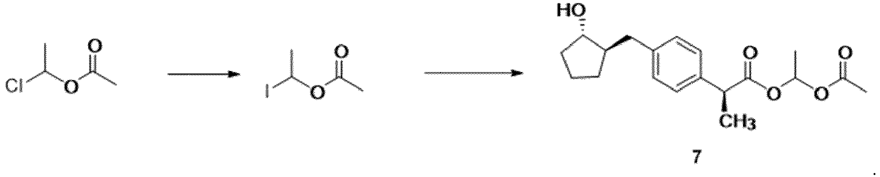

**Synthesis of 1-iodoethyl acetate:** 1-chloroethyl acetate (0.931 g, 6.56 mmol) was weighed into a dry 100 mL two-necked reaction flask, anhydrous NaI (983.2 mg, 5.84 mmol), TBAB (21.27 mg, 0.066 mmol), anhydrous CaCl₂ (262 mg, 2.36 mmol) and ethyl acetate (10 mL) were added, and the system was heated to 80 °C and refluxed for 3 h. The reaction flask was added with water and shaken, layers were separated, and the EA layer was washed with saturated salt solution, dried over anhydrous sodium sulfate and concentrated under reduced pressure to obtain 1-iodoethyl acetate (0.809 g) as a brown oil, which was directly used in the next step without purification.

**Synthesis of Compound 7:** at room temperature, Compound a (1.325 g, 5.34 mmol) was weighed and added to a dry 50 mL single-necked reaction flask, added with 10 mL of acetone and stirred to be dissolved. Then, 1-iodoethyl acetate (0.809 g, 3.78 mmol) was weighed and added to the above reaction flask and DBU (0.822 g, 5.40 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA = 4:1) to obtain the target product (1.631 g, 4.88 mmol) with a yield of 91.4%.

¹H NMR (500 MHz, *d6*-DMSO) *δ* 7.19(d, *J=* 8.0 Hz, 2H), 7.12(d, *J=* 8.0 Hz, 2H), 6.87-6.93(m, 1H), 4.25(s, 1H), 3.59-3.69(m, 2H), 2.78(dd, *J*= 13.5, 5.4 Hz, 1H), 2.08-2.11(m, 3H), 2.32(dd, J= 13.4, 9.4 Hz, 1H), 1.52-1.55(m, 3H)1.75-1.92(m, 2H), 1.39-1.70(m, 4H), 1.35(d, 3H, *J* = 7.1 Hz), 1.12-1.13(m, 1H).

### Example 8

### Compound 8 was synthesized according to the following reaction formula:

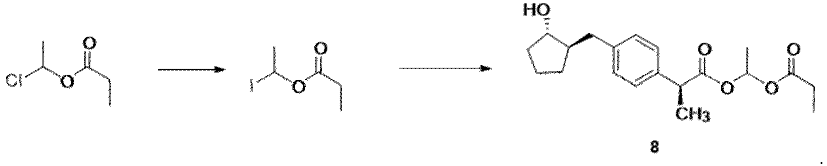

**Synthesis of 1-iodoethyl propionate:** 1-chloroethyl propionate (0.785 g, 5.77 mmol) was weighed into a dry 100 mL two-necked reaction flask, anhydrous NaI (764.4 mg, 5.1 mmol), TBAB (18.7 mg, 0.058 mmol), anhydrous CaCl₂ (230.6 mg, 2.08 mmol) and ethyl acetate (8 mL) were added, and the system was heated to 80 °C and refluxed for 3 h. The reaction flask was added with water and shaken, layers were separated, and the EA layer was washed with saturated salt solution, dried over anhydrous sodium sulfate and concentrated under reduced pressure to obtain 1-iodoethyl propionate (1.215 g) as a brown oil, which was directly used in the next step without purification.

**Synthesis of Compound 8:** at room temperature, Compound a (0.881 g, 3.55 mmol) was weighed and added to a dry 50 mL single-necked reaction flask, added with 10 mL of acetone and stirred to be dissolved. Then, 1-iodoethyl propionate (1.215 g, 5.33 mmol) was weighed and added to the above reaction flask and DBU (0.525 g, 3.45 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA = 4:1) to obtain the target product (1.097 g) with a yield of 88.7%.

¹H NMR (500MHz, *d6*-DMSO) *δ* 7.19(d, *J*= 8.0 Hz, 2H), 7.12(d, *J*= 8.0 Hz, 2H), 6.89-6.94(m, 1H), 4.25(s, 1H), 3.59-3.69(m, 2H), 2.78(dd, *J* = 13.5, 5.4 Hz, 1H), 2.41-2.35(m, 2H), 2.32(dd, *J=* 13.4, 9.4 Hz, 1H), 1.52-1.55(m, 3H)1.75-1.92(m, 2H), 1.39-1.70(m, 4H), 1.35(d, 3H, *J=* 7.1 Hz), 1.19-1.13(m, 3H)1.12-1.13(m, 1H).

### Example 9

### Compound 9 was synthesized according to the following reaction formula:

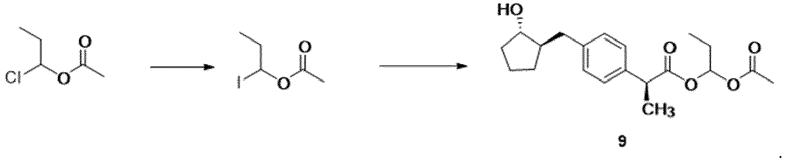

**Synthesis of 1-iodopropyl acetate:** 1-chloropropyl acetate (1.040 g, 7.65 mmol) was weighed into a dry 100 mL two-necked reaction flask, anhydrous NaI (1.02 g, 6.81 mmol), TBAB (24.81 mg, 0.077 mmol), anhydrous CaCl₂ (305.7 mg, 2.75 mmol) and ethyl acetate (12 mL) were added, and the system was heated to 80 °C and refluxed for 3 h. The reaction flask was added with water and shaken, layers were separated, and the EA layer was washed with saturated salt solution, dried over anhydrous sodium sulfate and concentrated under reduced pressure to obtain 1-iodopropyl acetate as a brown oil, which was directly used in the next step without purification.

**Synthesis of Compound 9:** at room temperature, Compound a (0.913 g, 3.68 mmol) was weighed and added to a dry 50 mL single-necked reaction flask, added with 10 mL of acetone and stirred to be dissolved. Then, 1-iodopropyl acetate (1.293 g, 5.67 mmol) was weighed and added to the above reaction flask and DBU (0.548 g, 3.60 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA = 4:1) to obtain the target product (1.086 g) with a yield of 84.8%.

¹H NMR (500 MHz, *d6*-DMSO) *δ* 7.19(d, *J* = 8.0 Hz, 2H), 7.12(d, *J* = 8.0 Hz, 2H), 6.77-6.82(m, 1H), 4.25(s, 1H), 3.59-3.69(m, 2H), 2.78(dd, *J* = 13.5, 5.4 Hz, 1H), , 2.32(dd, *J* = 13.4, 9.4 Hz, 1H), 2.27-2.29-(m, 3H), 1.75-1.92(m, 4H), 1.39-1.70(m, 4H), 1.35(d, 3H, *J* = 7.1 Hz), 1.12-1.13(m, 1H), 0.94-1.00(m, 3H)

### Example 10

### Compound 10 was synthesized according to the following reaction formula:

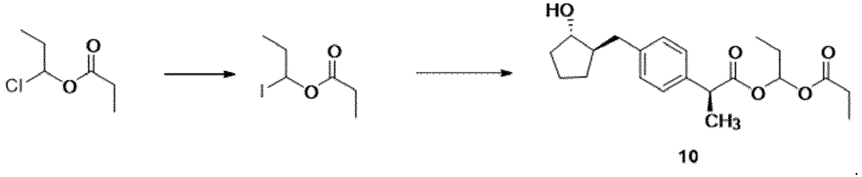

**Synthesis of 1-iodopropyl propionate:** 1-chloropropyl propionate (0.888 g, 5.92 mmol) was weighed into a dry 100 mL two-necked reaction flask, anhydrous NaI (789.7 mg, 5.27 mmol), TBAB (19 mg, 0.059 mmol), anhydrous CaCl₂ (236.6 mg, 2.13 mmol) and ethyl acetate (10 mL) were added, and the system was heated to 80 °C and refluxed for 3 h. The reaction flask was added with water and shaken, layers were separated, and the EA layer was washed with saturated salt solution, dried over anhydrous sodium sulfate and concentrated under reduced pressure to obtain 1-iodopropyl propionate (1.101 g) as a brown oil, which was directly used in the next step without purification.

**Synthesis of Compound** 10: at room temperature, Compound a (0.749 g, 3.02 mmol) was weighed and added to a dry 50 mL single-necked reaction flask, added with 10 mL of acetone and stirred to be dissolved. Then, 1-iodopropyl propionate (1.101 g, 4.55 mmol) was weighed and added to the above reaction flask and DBU (0.472 g, 3.10 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA = 4:1) to obtain the target product (0.848 g) with a yield of 77.5%.

¹H NMR (500 MHz, *d6*-DMSO) *δ* 7.19(d, *J* = 8.0 Hz, 2H), 6.77-6.83(m, 1H)7.12(d, *J* = 8.0 Hz, 2H), 4.25(s, 1H), 3.59-3.69(m, 2H), 2.78(dd, *J* = 13.5, 5.4 Hz, 1H), 2.33-2.41(m, 2H), 2.32(dd, *J* = 13.4, 9.4 Hz, 1H), 1.75-1.92(m, 4H), 1.39-1.70(m, 4H), 1.35(d, 3H, *J* = 7.1 Hz), 1.12-1.13(m, 4H), 0.95-0.99(m, 3H).

### Example 11

### Compound 11 was synthesized according to the following reaction formula:

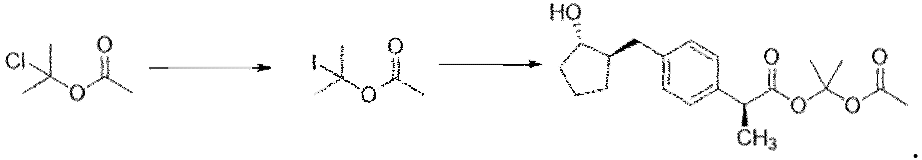

**Synthesis of 1-iodo-1-methyl-ethyl acetate:** 1-chloro-1-methyl-ethyl acetate (0.801 g, 5.89 mmol) was weighed into a dry 100 mL two-necked reaction flask, anhydrous NaI (785.4 mg, 5.24 mmol), TBAB (19 mg, 0.059 mmol), anhydrous CaCl₂ (235.3 mg, 2.12 mmol) and ethyl acetate (10 mL) were added, and the system was heated to 80 °C and refluxed for 3 h. The reaction flask was added with water and shaken, layers were separated, and the EA layer was washed with saturated salt solution, dried over anhydrous sodium sulfate and concentrated under reduced pressure to obtain 1-iodo-1-methyl-ethyl acetate (1.062 g) as a brown oil, which was directly used in the next step without purification.

**Synthesis of Compound 11:** at room temperature, Compound a (0.757 g, 3.05 mmol) was weighed and added to a dry 50 mL single-necked reaction flask, added with 10 mL of acetone and stirred to be dissolved. Then, 1-iodo-1-methyl-ethyl acetate (1.062 g, 4.66 mmol) was weighed and added to the above reaction flask and DBU (0.467 g, 3.07 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA = 4:1) to obtain the target product (0.846 g, 2.43 mmol) with a yield of 79.7%.

¹H NMR (500 MHz, *d6*-DMSO) *δ* 7.19(d, *J=* 8.0 Hz, 2H), 7.12(d, *J=* 8.0 Hz, 2H), 4.25(s, 1H), 3.59-3.69(m, 2H), 2.78(dd, *J* = 13.5, 5.4 Hz, 1H), 2.08-2.11(m, 3H), 2.32(dd, *J* = 13.4, 9.4 Hz, 1H), 1.75-1.92(m, 2H), 1.61-1.65(m, 6H), 1.39-1.70(m, 4H), 1.35(d, 3H, *J* = 7.1 Hz), 1.12-1.13(m, 1H).

### Example 12

### Compound 12 was synthesized according to the following reaction formula:

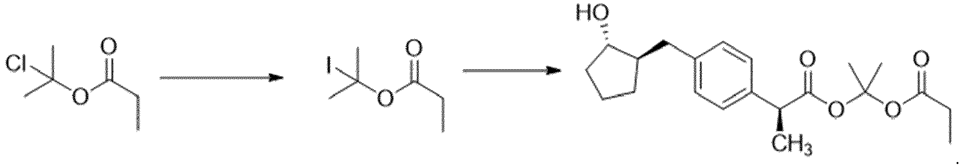

**Synthesis of 1-iodo-1-methyl-ethyl propionate:** 1-chloro-1-methyl-ethyl propionate (0.833 g, 5.55 mmol) was weighed into a dry 100 mL two-necked reaction flask, anhydrous NaI (740.4 mg, 4.94 mmol), TBAB (17.7 mg, 0.055 mmol), anhydrous CaCl₂ (222 mg, 2.0 mmol) and ethyl acetate (8 mL) were added, and the system was heated to 80 °C and refluxed for 3 h. The reaction flask was added with water and shaken, layers were separated, and the EA layer was washed with saturated salt solution, dried over anhydrous sodium sulfate and concentrated under reduced pressure to obtain 1-iodo-1-methyl-ethyl propionate (1.038 g) as a brown oil, which was directly used in the next step without purification.

**Synthesis of Compound 12:** at room temperature, Compound a (0.697 g, 2.81 mmol) was weighed and added to a dry 50 mL single-necked reaction flask, added with 10 mL of acetone and stirred to be dissolved. Then, 1-iodo-1-methyl-ethyl propionate (1.038 g, 4.29 mmol) was weighed and added to the above reaction flask and DBU (0.431 g, 2.83 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA = 4:1) to obtain the target product (0.623 g, 1.72 mmol) with a yield of 61.2%.

¹H NMR (500 MHz, *d6*-DMSO) δ 7.19(d, *J=* 8.0 Hz, 2H), 7.12(d, *J=* 8.0 Hz, 2H), 4.25(s, 1H), 3.59-3.69(m, 2H), 2.78(dd, *J* = 13.5, 5.4 Hz, 1H), 2.17-2.21(m, 2H), 2.32(dd, *J* = 13.4, 9.4 Hz, 1H), 1.75-1.92(m, 2H), 1.61-1.65(m, 6H), 1.39-1.70(m, 4H), 1.35(d, 3H, *J=* 7.1 Hz).

### Example 13

### Compound 13 was synthesized according to the following reaction formula:

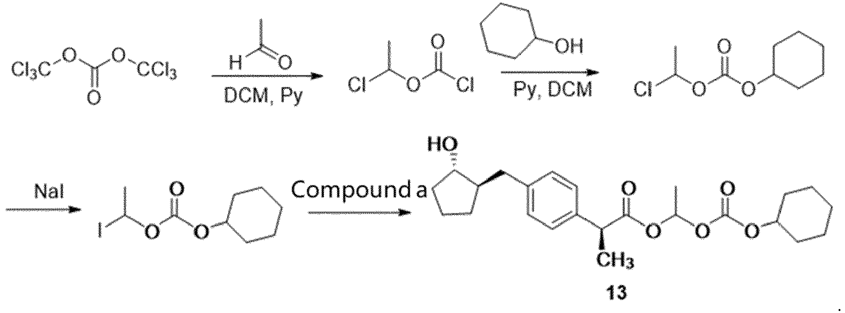

**Synthesis of 1-chloroethyl chloroformate:** triphosgene (10.01 g, 33.70 mmol) was weighed into a 100 mL three-necked reaction flask and added with 15 mL of anhydrous dichloromethane, and the reaction flask was purged with Ar three times, transferred to a cold trap of -20 °C and stirred continuously. Py (0.540 g, 6.83 mmol) was diluted with 5 mL of dichloromethane and then added to the reaction flask. Acetaldehyde (3.502 g, 79.46 mmol) was weighed and slowly added dropwise to the reaction flask. After dropwise addition, the temperature of the cold trap was set to -20 °C and the reaction was continued for 20 h. The reaction flask was pumped for 5 min using a water pump connected with a reaction flask containing an aqueous solution of KOH, and then transferred out of the cold trap. The solution was concentrated under reduced pressure for DCM to be removed and then distilled to obtain 1-chloroethyl chloroformate (3.912 g) as a colorless to pale yellow oil with a yield of 73.2%.

**Synthesis of 1-chloroethyl cyclohexyl carbonate:** 1-chloroethyl chloroformate (1.010 g, 6.38 mmol) was weighed into a dry two-necked reaction flask, added with 10 mL of anhydrous DCM and stirred continuously. Cyclohexanol (0.960 g, 9.56 mmol) was weighed and added to the above reaction flask. The reaction flask was transferred to an ice-water bath and stirred continuously. Pyridine (0.633 g, 7.96 mmol) was weighed and slowly added dropwise to the above reaction flask with a white solid appearing. After dropwise addition, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution and dried over anhydrous sodium sulfate. The resulting product was concentrated under reduced pressure to obtain 1-chloroethyl cyclohexyl carbonate as a colorless oil, which was directly used in the next step without purification.

**Synthesis of 1-iodoethyl cyclohexyl carbonate:** 1-chloroethyl cyclohexyl carbonate (1.008 g, 4.89 mmol) was weighed into a dry 100 mL two-necked reaction flask, anhydrous NaI (652 mg, 4.35 mmol), TBAB (15.8 mg, 0.049 mmol), anhydrous CaCl₂ (195.4 mg, 1.76 mmol) and ethyl acetate (10 mL) were added, and the system was heated to 80 °C and refluxed for 3 h. The reaction flask was added with water and shaken, layers were separated, and the EA layer was washed with saturated salt solution, dried over anhydrous sodium sulfate and concentrated under reduced pressure to obtain 1-iodoethyl cyclohexyl carbonate as a brown oil, which was directly used in the next step without purification.

**Synthesis of Compound 13:** at room temperature, Compound a (0.462 g, 1.86 mmol) was weighed and added to a dry 50 mL single-necked reaction flask, added with 10 mL of acetone and stirred to be dissolved. Then, 1-iodoethyl cyclohexyl carbonate (0.697 g, 2.81 mmol) was weighed and added to the above reaction flask and DBU (0.274 g, 1.80 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA = 4:1) to obtain the target product (0.598 g, 1.43 mmol) with a yield of 76.9%.

¹H NMR (500 MHz, *d6*-DMSO) *δ* 7.19(d, *J=* 8.0 Hz, 2H), 7.12(d, *J* = 8.0 Hz, 2H), 6.77-6.82(m, 1H), 4.60-4.67(m, 1H)4.25(s, 1H), , 3.59-3.69(m, 2H), 2.78(dd, *J* = 13.5, 5.4 Hz, 1H), 2.32(dd, *J* = 13.4, 9.4 Hz, 1H), 1.75-1.92(m, 5H), 1.39-1.70(m, 13H), 1.35(d, 3H, *J* = 7.1 Hz), 1.12-1.13(m, 1H).

### Example 14

### Compound 14 was synthesized according to the following reaction formula:

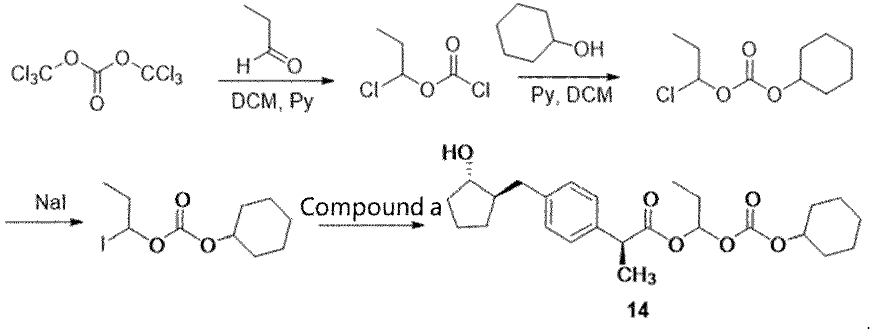

**Synthesis of 1-chloropropyl chloroformate:** triphosgene (10.01 g, 33.70 mmol) was weighed into a 100 mL three-necked reaction flask and added with 15 mL of anhydrous dichloromethane, and the reaction flask was purged with Ar three times, transferred to a cold trap of -20 °C and stirred continuously. Py (0.542 g, 6.83 mmol) was diluted with 5 mL of dichloromethane and then added to the reaction flask. N-propionaldehyde (4.304 g, 79.46 mmol) was weighed and slowly added dropwise to the reaction flask. After dropwise addition, the temperature of the cold trap was set to -20 °C and the reaction was continued for 20 h. The reaction flask was pumped for 5 min using a water pump connected with a reaction flask containing an aqueous solution of KOH, and then transferred out of the cold trap. The solution was concentrated under reduced pressure for DCM to be removed and then distilled to obtain 1-chloropropyl chloroformate (3.910 g) as a colorless to pale yellow oil with a yield of 73.2%.

**Synthesis of 1-chloropropyl cyclohexyl carbonate:** 1-chloropropyl chloroformate (1.025 g, 6.57 mmol) was weighed into a dry two-necked reaction flask, added with 10 mL of anhydrous DCM and stirred continuously. Cyclohexanol (0.960 g, 9.56 mmol) was weighed and added to the above reaction flask. The reaction flask was transferred to an ice-water bath and stirred continuously. Pyridine (0.631 g, 7.96 mmol) was weighed and slowly added dropwise to the above reaction flask with a white solid appearing. After dropwise addition, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution and dried over anhydrous sodium sulfate. The resulting product was concentrated under reduced pressure to obtain 1-chloropropyl cyclohexyl carbonate (1.008 g) as a colorless oil, which was directly used in the next step without purification.

**Synthesis of 1-iodopropyl cyclohexyl carbonate:** 1-chloropropyl cyclohexyl carbonate (1.008 g, 4.58 mmol) was weighed into a dry 100 mL two-necked reaction flask, anhydrous NaI (611 mg, 4.08 mmol), TBAB (14.8 mg, 0.046 mmol), anhydrous CaCl₂ (183.2 mg, 1.65 mmol) and ethyl acetate (12 mL) were added, and the system was heated to 80 °C and refluxed for 3 h. The reaction flask was added with water and shaken, layers were separated, and the EA layer was washed with saturated salt solution, dried over anhydrous sodium sulfate and concentrated under reduced pressure to obtain 1-iodopropyl cyclohexyl carbonate as a brown oil, which was directly used in the next step without purification.

**Synthesis of Compound 14 by the following reaction formula:** at room temperature, Compound a (0.625 g, 2.52 mmol) was weighed and added to a dry 50 mL single-necked reaction flask, added with 10 mL of acetone and stirred to be dissolved. Then, 1-iodopropyl cyclohexyl carbonate (1.179 g, 3.78 mmol) was weighed and added to the above reaction flask and DBU (383.6 mg, 2.52 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA= 4:1) to obtain the target product (1.054 g) with a yield of 79.8%.

¹H NMR (500 MHz, *d6*-DMSO) *δ* 7.19(d, *J* = 8.0 Hz, 2H), 7.12(d, *J* = 8.0 Hz, 2H), 6.77-6.82(m, 1H), 4.60-4.67(m, 1H)4.25(s, 1H), , 3.59-3.69(m, 2H), 2.78(dd, *J* = 13.5, 5.4 Hz, 1H), 2.32(dd, *J* = 13.4, 9.4 Hz, 1H), 1.75-1.92(m, 8H), 1.39-1.70(m, 13H)1.35(d, 3H, *J* = 7.1 Hz), 1.12-1.13(m, 1H).

### Example 15

### Compound 15 was synthesized according to the following reaction formula:

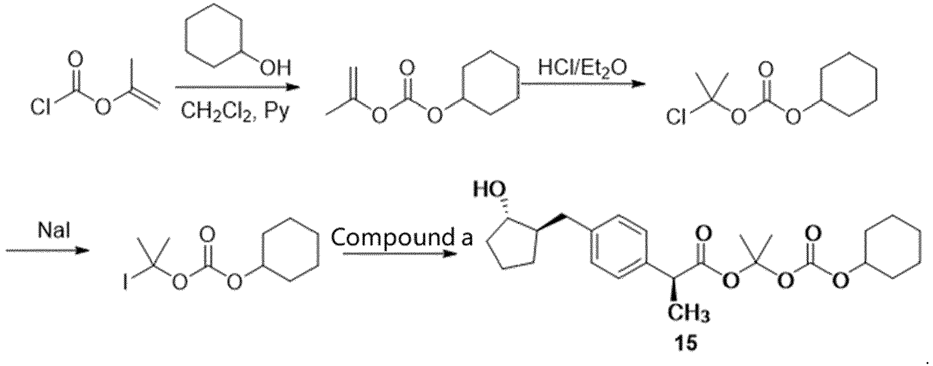

**Synthesis of 1-chloro-1-methyl-ethyl cyclohexyl carbonate:** 2-propenyl chloroformate (1.006 g, 8.30 mmol) was weighed into a single-necked reaction flask, added with 20 mL of anhydrous dichloromethane and stirred continuously. Cyclohexanol (0.837 g, 8.30 mmol) was weighed and added to the above reaction flask and the reaction flask was transferred to an ice bath and stirred. Pyridine (0.690 g, 8.72 mmol) diluted with 5 mL of dry CH₂Cl₂ was slowly added dropwise and then the system was reacted at room temperature for 2 h and poured into 20 mL of ice water. Organic phases were dried over anhydrous sodium sulfate and filtered. The filtrate was evaporated to dryness to obtain an oily liquid. The liquid was added with 10 mL of ether. Then, 50 mL of 4N hydrochloric acid/ether was slowly added dropwise. After addition, the system was stirred overnight at room temperature and concentrated under reduced pressure to obtain 1-chloro-1-methyl-ethyl cyclohexyl carbonate (1.589 g) as a colorless liquid, which was directly used in the next step without purification.

**Synthesis of 1-iodo-methyl-ethyl cyclohexyl carbonate:** 1-chloro-1-methyl-ethyl cyclohexyl carbonate (1.589 g, 7.22 mmol) was weighed into a dry 100 mL two-necked reaction flask, anhydrous NaI (963.8 mg, 6.43 mmol), TBAB (23.2 mg, 0.072 mmol), anhydrous CaCl₂ (288.6 mg, 2.6 mmol) and ethyl acetate (15 mL) were added, and the system was heated to 80 °C and refluxed for 3 h. The reaction flask was added with water and shaken, layers were separated, and the EA layer was washed with saturated salt solution, dried over anhydrous sodium sulfate and concentrated under reduced pressure to obtain 1-iodo-1-methyl-ethyl cyclohexyl carbonate (1.938 g) as a brown oil, which was directly used in the next step without purification.

**Synthesis of Compound 15:** at room temperature, Compound a (1.027 g, 4.14 mmol) was weighed and added to a dry 50 mL single-necked reaction flask, added with 10 mL of acetone and stirred to be dissolved. Then, 1-iodo-1-methyl-ethyl cyclohexyl carbonate (1.938 g, 6.21 mmol) was weighed and added to the above reaction flask and DBU (0.632 g, 4.15 mmol) was weighed and slowly added dropwise to the reaction flask. After dropwise addition, the reaction was stirred at room temperature overnight. Water (10 mL) and ethyl acetate (30 mL) were added to the reaction flask, layers were separated, and the organic layer was washed with 5% NaHCO₃, water and saturated salt solution, dried over anhydrous sodium sulfate, concentrated under reduced pressure and subjected to flash column chromatography (PE/EA = 4:1) to obtain the target product (1.567 g) with a yield of 72.2%.

¹H NMR (500MHz, *d6*-DMSO) *δ* 7.19(d, *J* = 8.0 Hz, 2H), 7.12(d, *J* = 8.0 Hz, 2H), 4.60-4.67(m, 1H)4.25(s, 1H), , 3.59-3.69(m, 2H), 2.78(dd, *J* = 13.5, 5.4 Hz, 1H), 2.32(dd, *J* = 13.4, 9.4 Hz, 1H), 1.75-1.92(m, 6H), 1.39-1.70(m, 16H), 1.35(d, 3H, *J* = 7.1 Hz), 1.12-1.13(m, 1H).

### Example 16

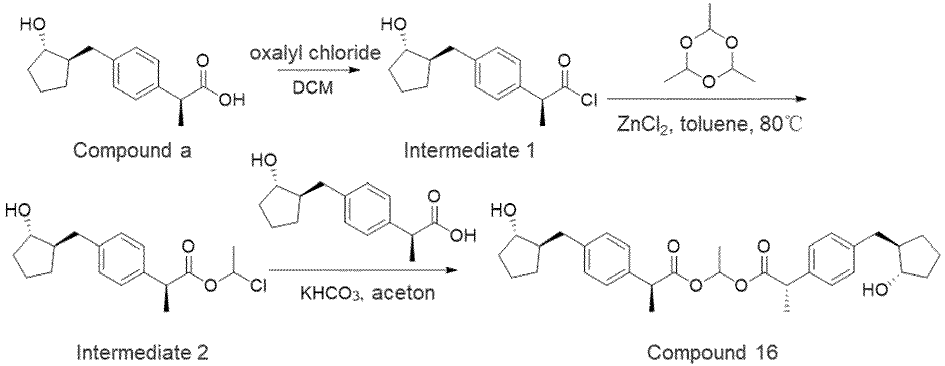

### Synthesis of Intermediate 1

Compound a (1.2 g, 4.83 mmol) was weighed into a 50 mL pre-dried two-necked reaction flask, 10 mL of anhydrous DCM was added, and the reaction flask was purged with N₂ two times while being stirred. The reaction flask was transferred to an ice bath and stirred continuously. Five drops of DMF were added and oxalyl chloride (0.918 g, 7.24 mmol) was slowly added dropwise to the reaction flask. After dropwise addition, the system was reacted for 2 h in the ice bath. The reaction was concentrated under reduced pressure for the solvent to be removed to obtain a yellow oil (1.13 g), which was directly used in the next step without further purification.

### Synthesis of Intermediate 2

Under N₂ protection, Intermediate 1 (1.13 g, 4.24 mmol) just prepared was placed in a 50 mL two-necked reaction flask and anhydrous ZnCl₂ (58 mg, 0.424 mmol) was added to the reaction flask. Anhydrous toluene (20 mL) was added to the reaction flask and the reaction flask was transferred to an ice bath and stirred continuously. Paraldehyde (209.2 mg, 1.58 mmol) was weighed, diluted with 5 mL of toluene and slowly added to the above reaction flask. After dropwise addition, the reaction was transferred to 80 °C, heated and reacted for 5 h. 10 mL of water was added to the reaction flask, layers were separated, and the DCM layer was washed with an aqueous solution of 5% NaHCO₃ twice to a pH of 8-9, washed with water and saturated salt solution in sequence, dried over anhydrous sodium sulfate and concentrated under reduced pressure to obtain a crude product of Intermediate 2 as a yellow oil (0.91 g), which is directly used in the next step without purification.

### Synthesis of Compound 16

Compound a (0.54 g, 2.17 mmol) and KHCO₃ (0.38 g, 3.8 mmol) were weighed into a 50 mL single-necked reaction flask, added with 10 mL of acetone and stirred continuously. Then, Intermediate 2 (0.91 g, 2.93 mmol) just prepared was added to the reaction flask and the reaction was heated to 60 °C and carried out for 6 h. DCM and water were added to the reaction, layers were separated, and the DCM layer was washed with 5% NaHCO₃, water and saturated salt solution, dried, concentrated and subjected to flash column chromatography (PE/EA = 4:1) to obtain a yellow oil (0.427 g) with a yield of 37.6%.

¹H NMR (500 MHz, *d6*-DMSO) δ 7.19(d, *J* = 8.0 Hz, 4H), 7.12(d, *J=* 8.0 Hz, 4H), 6.87-6.93(m, 1H), 4.25(s, 2H), 3.59-3.69(m, 4H), 2.78(dd, *J* = 13.5, 5.4 Hz, 2H), 2.32(dd, *J* = 13.4, 9.4 Hz, 2H), 1.52-1.55(m, 6H)1.75-1.92(m, 4H), 1.39-1.70(m, 8H), 1.35(d, 3H, *J* = 7.1 Hz), 1.12-1.13(m, 2H).

### Test Example 1: Study on metabolism of the compound of the present application in human plasma

(1) 40 mM pure acetonitrile stock solutions of Compound 1, Compound 7 and Compound a were prepared;
(2) the stock solution (25 µL) of Compound a was mixed with 1 mL of human plasma and vortexed for 30s, a sample (200 µL) was added to 800 µL of acetonitrile to precipitate proteins and vortexed for 1 min, and then the reaction was stopped as a control of an active metabolite of loxoprofen; and 40 mM stock solutions of Compound 1 and Compound 7 were diluted 200 times as prodrug controls;
(4) the pure acetonitrile stock solution (100 µL) of each of Compound 1 and Compound 7 was mixed with 4 mL of human plasma, vortexed for 30s, and oscillated at 200 rpm in an oscillating water bath heater with a constant temperature of 37 °C;
(5) samples (200 µL) were taken at different time points (0, 15, 30, 60 and 120 min) with three samples at each time point, added with 800 µL of acetonitrile to precipitate proteins and vortexed for 1 min, and then the reaction was stopped; and a blank plasma control was obtained in the same manner;
(6) the samples were centrifuged at 12000 rpm for 10 min at 4 °C, the supernatant was taken and injected at a volume of 30 µL (through a filter membrane), and changes of a peak area were recorded;
(7) hydrolysis rates of Compound 1 and Compound 7 were observed and analyzed.

Experimental results are shown in Table 1.

**Table 1 Metabolism of Compound 1 and Compound 7**

| Compound | Remaining Amount of the Prodrug Compound (%) | Amount of the Active Metabolite Produced (%) |
|---|---|---|
| 1 | 35.41%±1.83% | 53.40%±3.26% |
| 7 | 21.41%±0.49% | 73.94%±3.66% |

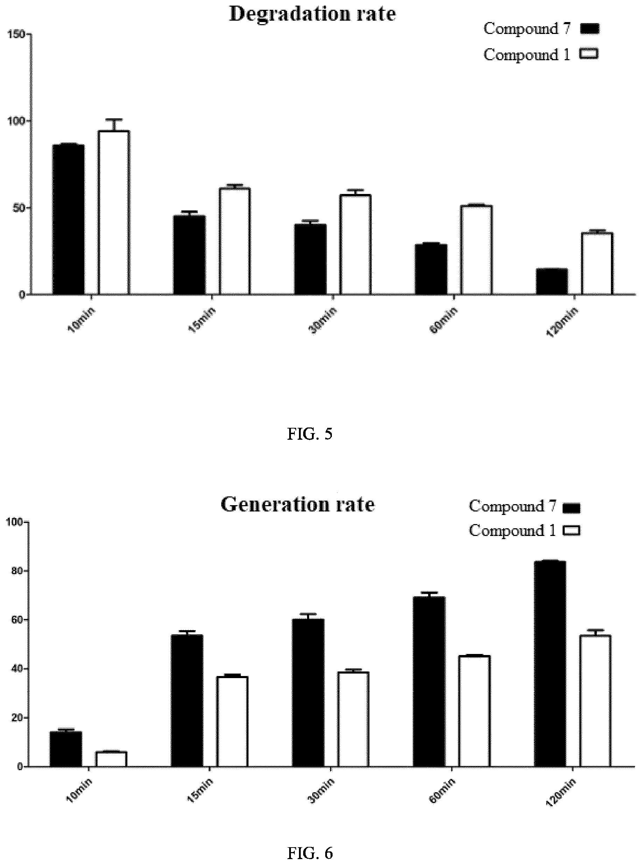

As can be seen from test results, the metabolic rate of Compound 7 is slightly faster than that of Compound 1 without a significant difference and the two compounds can both be rapidly transformed into the active metabolite in in vitro human plasma to exert their pharmacologically active effects (as shown in FIGs. 5 and 6).

## Claims

1. A compound represented by Structural Formula (I) or a racemate, stereoisomer or pharmaceutically acceptable salt or solvate thereof, 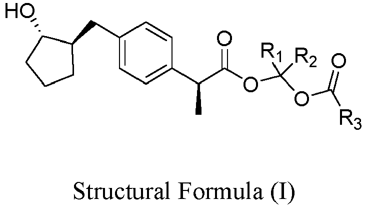 wherein R₁, R₂ and R₃ are the same or different and each independently selected from hydrogen, C₁₋₄₀ alkyl, C₂₋₄₀ alkenyl, C₂₋₄₀ alkynyl, C₁₋₄₀ alkoxy, C₃₋₄₀ cycloalkyl, C₃₋₄₀ cycloalkyloxy, 3- to 20-membered heterocyclic groups, C₆₋₂₀ aryl, 5- to 20-membered heteroaryl or 3- to 20-membered heterocyclic groups substituted with one, two or more Ra; wherein the one, two or more Ra are the same or different and each independently selected from halogen, C₁₋₄₀ alkyl, C₁₋₄₀ alkoxy or C6-20 arylacyl.

2. The compound according to claim 1, wherein R₁, R₂ and R₃ are the same or different and each independently selected from hydrogen, C₁₋₂₀ alkyl, C₂₋₂₀ alkenyl, C₂₋₂₀ alkynyl, C₁₋₂₀ alkoxy, C₃₋₂₀ cycloalkyl, C₃₋₂₀ cycloalkyloxy, 5- to 10-membered heterocyclic groups, C₆₋₁₄ aryl, 5- to 14-membered heteroaryl or 5- to 14-membered heterocyclic groups substituted with one, two or more Ra; wherein the one, two or more Ra are the same or different and each independently selected from halogen, C₁₋₂₀ alkyl, C₁₋₂₀ alkoxy or C₆₋₂₀ arylacyl.

3. The compound according to claim 1, wherein R₁, R₂ and R₃ are the same or different and each independently selected from hydrogen, C₁₋₈ alkyl, C₂₋₈ alkenyl, C₂₋₈ alkynyl, C₁₋₈ alkoxy, C₃₋₈ cycloalkyl, C₃₋₈ cycloalkyloxy, 5- to 10-membered heterocyclic groups, C₆₋₁₀ aryl, 5- to 10-membered heteroaryl or 5- to 10-membered heterocyclic groups substituted with one, two or more Ra; wherein the one, two or more Ra are the same or different and each independently selected from C₆₋₁₀ arylacyl.

4. The compound according to claim 1, wherein R₁, R₂ and R₃ are the same or different and each independently selected from hydrogen, C₁₋₆ alkyl, C₂₋₆ alkenyl, C₂₋₆ alkynyl, C₁₋₆ alkoxy, C₃₋₆ cycloalkyl, C₃₋₆ cycloalkyloxy, 5- to 8-membered heterocyclic groups, C₆₋₈ aryl, 5- to 8-membered heteroaryl or 5- to 8-membered heterocyclic groups substituted with one, two or more Ra; wherein the one, two or more Ra are the same or different and each independently selected from C₆₋₁₀ arylacyl, for example, benzoyl.

5. The compound according to claim 1, wherein R₁ is selected from hydrogen, methyl, ethyl, isopropyl, isobutyl, t-butyl, cyclopropyl, cyclobutyl, cyclopentyl or cyclohexyl; R₂ is selected from hydrogen, methyl, ethyl, isopropyl, isobutyl, t-butyl, cyclopropyl, cyclobutyl, cyclopentyl or cyclohexyl; and R₃ is selected from methyl, ethyl, isopropyl, t-butyl, isobutyl, cyclopropyl, cyclobutyl, cyclopentyl, cyclohexyl, methoxy, ethoxy, isopropoxy, t-butoxy, isobutoxy, cyclopropoxy, cyclobutoxy, cyclopentyloxy, cyclohexyloxy or 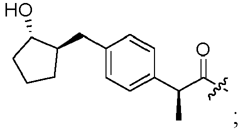 wherein 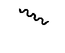 represents a linkage site.

6. The compound according to claim 1, wherein the compound represented by Formula (I) is selected from the following structures:

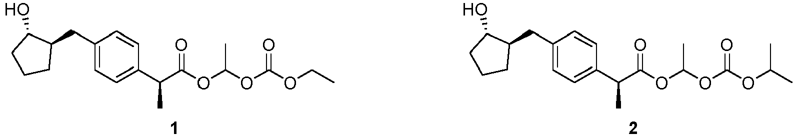

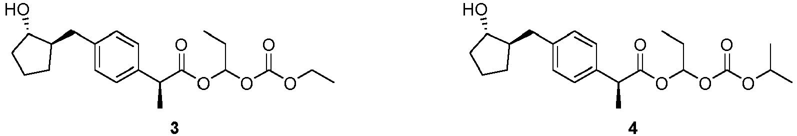

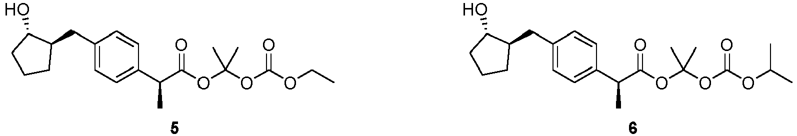

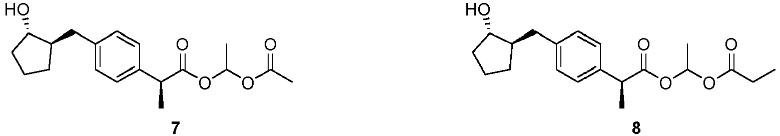

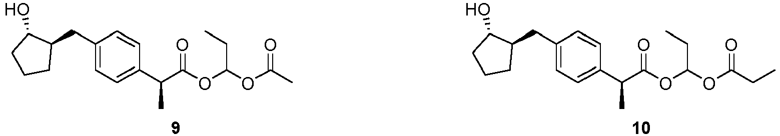

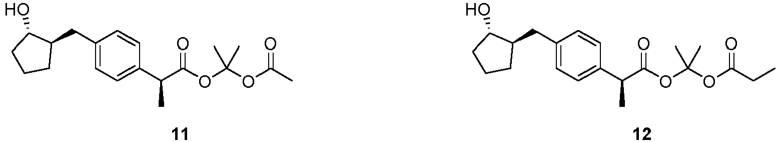

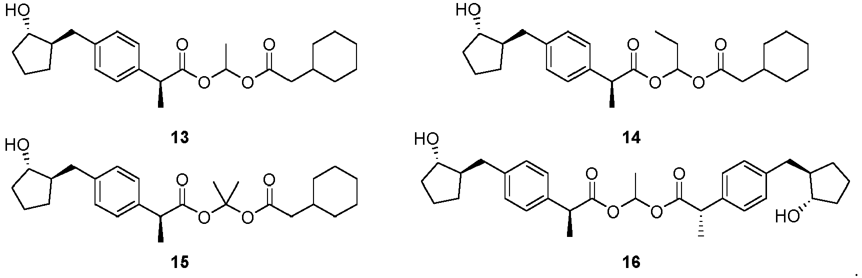

7. A method for preparing the compound according to any one of claims 1 to 6, comprising:
reacting Compound a with Compound b to give the compound represented by Formula (I): 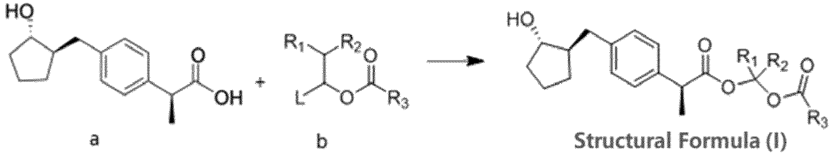
wherein R₁, R₂ and R₃ each independently have the definitions as described above;
L is selected from leaving groups, for example, halogen and hydroxyl; and
Compound a is an active metabolite of loxoprofen, that is, (S)-2-(4-(((1R, 2S)-2-hydroxycyclopentyl)methyl)phenyl)propionic acid;
preferably, Compound 2 is selected from the following Compound 3 or Compound 4: 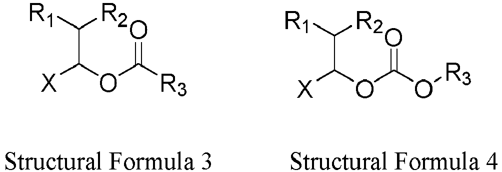
wherein R₁, R₂ and R₃ each independently have the definitions as described above; and
X is selected from chlorine, bromine or iodine.

8. The method according to claim 7, wherein the method may be performed in the presence of an organic solvent; wherein the organic solvent may be selected from at least one of: acetone, dimethylsulfoxide, N,N-dimethylformamide; ethers such as ethyl propyl ether, n-butyl ether, methyl phenyl ether, ethyl phenyl ether, cyclohexyl methyl ether, dimethyl ether, diethyl ether, dimethyl glycol, diphenyl ether, dipropyl ether, diisopropyl ether, di-n-butyl ether, diisobutyl ether, diisopentyl ether, ethylene glycol dimethyl ether, isopropyl ethyl ether, methyl t-butyl ether, tetrahydrofuran, methyl tetrahydrofuran, dioxane, dichlorodiethyl ether and polyethers of ethylene oxide and/or propylene oxide; aliphatic, cycloaliphatic or aromatic hydrocarbons such as pentane, hexane, heptane, octane and nonane; classes that may be substituted with fluorine and chlorine atoms, such as dichloromethane, chloroform, carbon tetrachloride, fluorobenzene, chlorobenzene or dichlorobenzene; cyclohexane, methylcyclohexane, petroleum ether, octane, benzene, toluene, chlorobenzene, bromobenzene, xylene; and esters such as methyl acetate, ethyl acetate, butyl acetate, isobutyl acetate, dimethyl carbonate, dibutyl carbonate or vinyl carbonate;
preferably, the method may be performed in the presence of an acid-binding agent such as a base; wherein the base may be an organic base or an inorganic base; the inorganic base may be selected from at least one of a hydride, hydroxide, alkoxide, acetate, fluoride, phosphate, carbonate and bicarbonate of an alkali metal or an alkaline-earth metal, and a preferred base is sodium amino, sodium hydride, lithium diisopropylamino, sodium methoxide, potassium tert-butoxide, sodium hydroxide, potassium hydroxide, sodium acetate, sodium phosphate, potassium phosphate, potassium fluoride, cesium fluoride, sodium carbonate, potassium carbonate, potassium bicarbonate, sodium bicarbonate and cesium carbonate; and the organic base may be selected from at least one of tertiary amines, substituted or unsubstituted pyridines and substituted or unsubstituted triethylamine, trimethylamine, N,N-diisopropylethylamine, tri-n-propylamine, tri-n-butylamine, tri-n-hexylamine, tricyclohexylamine, N-methylcyclohexylamine, N-methylpyrrolidine, N-methylpiperidine, N-ethylpiperidine, N,N-dimethylaniline, N-methylmorpholine, pyridine, 2-, 3- or 4-methylpyridine, 2-methyl-5-ethylpyridine, 2,6-dimethylpyridine, 2,4,6-trimethylpyridine, 4-dimethylaminopyridine, quinoline, methylquinoline, N,N,N,N-tetramethyl ethylene diamine, N,N-dimethyl-1,4-diazacyclohexane, N,N-diethyl-1,4-diazacyclohexane, 1,8-bis(dimethylamino)naphthalene, diazabicyclooctane (DABCO), diazabicyclononane (DBN), diazabicycloundecane (DBU), butyl imidazole or methyl imidazole;
preferably, the method may be performed in the presence of a catalyst such as a phase transfer catalyst; wherein the catalyst may be selected from tetrabutylammonium bromide (TBAB), tetrabutylammonium chloride (TBAC), tetrabutylammonium iodide (TBAI), potassium iodide, sodium iodide or 18-crown ether-6;
preferably, the method is performed at a reaction temperature of -5-80 °C, for example, 0-50 °C;
preferably, the method is performed for 0.5-24 h, for example, 1-12 h.

9. An application of the compound represented by Formula (I) according to any one of claims 1 to 6, or the racemate, stereoisomer or pharmaceutically acceptable salt or solvate thereof to preparation of a nonsteroidal anti-inflammatory drug;
wherein the nonsteroidal anti-inflammatory drug is preferably used for anti-inflammation and/or analgesia of rheumatoid arthritis, low back pain, migraine, neuralgia, periarthritis of shoulder or osteoarthritis, neck-shoulder-wrist syndromes, analgesia and/or anti-inflammation after surgery, trauma or tooth extraction, and antipyretic and/or analgesia of acute upper respiratory tract inflammation.

10. A pharmaceutical composition, comprising the compound represented by Structural Formula (1) according to any one of claims 1 to 6, or the racemate, stereoisomer or pharmaceutically acceptable salt or solvate thereof; wherein a dosage form of the pharmaceutical composition comprises a tablet, a capsule, granules, an eye drop, a gel, latex, cream, an ointment, cataplasm, a gel paste, a solution injection and an emulsion injection.
